# EUROPEAN PATENT SPECIFICATION

(11) **EP 1 798 293 B1**
(45) Date of publication and mention of the grant of the patent: **20.05.2015**
(21) Application number: 06025433.1
(22) Date of filing: 08.12.2006
(51) Int. Cl.: C12Q 1/68

(54) **Gene set used for examination of colon cancer**
Gensatz zur Untersuchung von Kolonkrebs
Groupe de gènes utilisés pour l'examen du cancer du colon

(30) Priority: 14.12.2005 JP 2005360974
(43) Date of publication of application: 20.06.2007
(73) Proprietor: CANON KABUSHIKI KAISHA, Tokyo (JP); President of National Cancer Center, Chuo-ku Tokyo (JP)
(72) Inventor: Ishii, Mie, Ohta-ku Tokyo (JP); Yamamoto, Nobuko, Ohta-ku Tokyo (JP); Kawaguchi, Masahiro, Ohta-ku Tokyo (JP); Okabe, Tetsuo, Ohta-ku Tokyo (JP); Sasaki, Hiroki, 5-1-1 Tsukiji Chuo-ku Tokyo (JP); Yajima, Satoshi, 5-1-1 Tsukiji Chuo-ku Tokyo (JP); Matsumura, Yasuhiro, 6-5-1 Kashiwanoha Kashiwa-shi Chiba-ken (JP); Matsushita, Hisayuki, 6-5-1 Kashiwanoha Kashiwa-shi Chiba-ken (JP); Tunoda, Hiroyuki, Kodaira-shi Tokyo (JP); Harada, Kunio, Hachioji-shi Tokyo (JP)
(74) Representative: WESER & Kollegen

(56) References cited:
- RECHRECHE HOCINE ET AL: "pap, reg Ialpha and reg Ibeta mRNAs are concomitantly up-regulated during human colorectal carcinogenesis" INTERNATIONAL JOURNAL OF CANCER, vol. 81, no. 5, 31 May 1999 (1999-05-31), pages 688-694, XP002430541 ISSN: 0020-7136
- MCIVER C M ET AL: "Dipeptidase 1: a candidate tumor-specific molecular marker in colorectal carcinoma" CANCER LETTERS, NEW YORK, NY, US, vol. 209, no. 1, 8 June 2004 (2004-06-08), pages 67-74, XP002368418 ISSN: 0304-3835
- DI RENZO M F ET AL: "Overexpression and amplification of the met/HGF receptor gene during the progression of colorectal cancer." CLINICAL CANCER RESEARCH : AN OFFICIAL JOURNAL OF THE AMERICAN ASSOCIATION FOR CANCER RESEARCH FEB 1995, vol. 1, no. 2, February 1995 (1995-02), pages 147-154, XP002430542 ISSN: 1078-0432
- MACADAM R C A ET AL: "Death from early colorectal cancer is predicted by the presence of transcripts of the REG gene family" BRITISH JOURNAL OF CANCER, vol. 83, no. 2, July 2000 (2000-07), pages 188-195, XP002430543 ISSN: 0007-0920
- OTTE JAN-MICHEL ET AL: "Functional expression of HGF and its receptor in human colorectal cancer" DIGESTION, vol. 61, no. 4, 2000, pages 237-246, XP009082542 ISSN: 0012-2823
- HISCOX STEPHEN E ET AL: "Expression of the HGF/SF receptor, c-met, and its ligand in human colorectal cancers" CANCER INVESTIGATION, vol. 15, no. 6, 1997, pages 513-521, XP009082541 ISSN: 0735-7907
- LIU C ET AL: "OVEREXPRESSION OF C-MET PROTO-ONCOGENE BUT NOT EPIDERMAL GROWTH FACTOR RECEPTOR OR C-ERBB-2 IN PRIMARY HUMAN COLORECTAL CARCINOMAS" ONCOGENE, vol. 7, no. 1, 1992, pages 181-186, XP009082599 ISSN: 0950-9232
- FARID E AHMED ET AL: "Improved Methods for Extracting RNA from Exfoliated Human Colonocytes in Stool and RT-PCR Analysis", DIGESTIVE DISEASES AND SCIENCES, KLUWER ACADEMIC PUBLISHERS-PLENUM PUBLISHERS, NE, vol. 49, no. 11-12, 1 November 2004 (2004-11-01), pages 1889-1898, XP019237033, ISSN: 1573-2568, DOI: 10.1007/S10620-004-9589-9

### SEQUENCE LISTING

<110> CANON KABUSHIKI KAISHA PRESIDENT OF NATIONAL CANCER CENTER
<120> Gene set used for examination of colon cancer
<130> 10041420EP01
<150> JP2005-360974
   <151> 2005-12-14
<160> 171
<170> PatentIn Ver. 2.1
<210> 1
   <211> 60
   <212> DNA
   <213> Artificial Sequence
<220>
   <223> Description of Artificial Sequence:probe
<400> 1
   ttcccccaac ctgaccacct cattcttatc tttcttctgt ttcttcctcc ccgctgtcat 60
<210> 2
   <211> 60
   <212> DNA
   <213> Artificial Sequence
<220>
   <223> Description of Artificial Sequence:probe
<400> 2
   gaccatctct ccaactcaac tcaacctgga cactctcttc tctgctgagt ttgccttgtt 60
<210> 3
   <211> 53
   <212> DNA
   <213> Artificial Sequence
<220>
   <223> Description of Artificial Sequence:probe
<400> 3
   gaggcatgtc tggttcggcg agagcatgac cgatggattc cagttcgagt atg 53
<210> 4
   <211> 56
   <212> DNA
   <213> Artificial Sequence
<220>
   <223> Description of Artificial Sequence:probe
<400> 4
   catgacaact gccgggaggg ccacgcaggt cgtggtcacc tgccagcgac tgtctc 56
<210> 5
   <211> 60
   <212> DNA
   <213> Artificial Sequence
<220>
   <223> Description of Artificial Sequence:probe
<400> 5
   caggggcttc tagctgactc gcacagggat tctcacaata gccgatatca gaatttgtgt 60
<210> 6
   <211> 52
   <212> DNA
   <213> Artificial Sequence
<220>
   <223> Description of Artificial Sequence:probe
<400> 6
   cagatgccag gagccctgct gcccacatgc aaggaccagc atctcctgag ag 52
<210> 7
   <211> 56
   <212> DNA
   <213> Artificial Sequence
<220>
   <223> Description of Artificial Sequence:probe
<400> 7
   tattgccgaa ccggccgttg tgctacccgt gagtccctct ccggggtgtg tgaaat 56
<210> 8
   <211> 60
   <212> DNA
   <213> Artificial Sequence
<220>
   <223> Description of Artificial Sequence:probe
<400> 8
   atctgtatga caacccggga tcgtttgcaa gtaactgaat ccattgcgac attgtgaagg 60
<210> 9
   <211> 60
   <212> DNA
   <213> Artificial Sequence
<220>
   <223> Description of Artificial Sequence:probe
<400> 9
   acaggatcgt atcatatact cgagacttac cgcatattac agtggatcga ttagtgtcaa 60
<210> 10
   <211> 54
   <212> DNA
   <213> Artificial Sequence
<220>
   <223> Description of Artificial Sequence:probe
<400> 10
   cagcattcct gcactaacgg caactctacg atgtgtccgt gaccctcaga gatc 54
<210> 11
   <211> 60
   <212> DNA
   <213> Artificial Sequence
<220>
   <223> Description of Artificial Sequence:probe
<400> 11
   acaaacccga aaagagtgtg agctaactag tttccaaagc ggagacttcc gacttcctta 60
<210> 12
   <211> 60
   <212> DNA
   <213> Artificial Sequence
<220>
   <223> Description of Artificial Sequence:probe
<400> 12
   acttgcacgt gtccctgaat tccgctgact ctaatttatg aggatgccga actctgatgg 60
<210> 13
   <211> 53
   <212> DNA
   <213> Artificial Sequence
<220>
   <223> Description of Artificial Sequence:probe
<400> 13
   ggaactgacg cagctacgcc atgaactgga gcggcagaac aatgaatacc aag 53
<210> 14
   <211> 53
   <212> DNA
   <213> Artificial Sequence
<220>
   <223> Description of Artificial Sequence:probe
<400> 14
   ccgcggccat ggcgtacgcc tatctcttca agtacatcat aatcggcgac aca 53
<210> 15
   <211> 60
   <212> DNA
   <213> Artificial Sequence
<220>
   <223> Description of Artificial Sequence:probe
<400> 15
   tctgttgccg aaatagctgg tcctttttcg ggagttagat gtatagagtg tttgtatgta 60
<210> 16
   <211> 54
   <212> DNA
   <213> Artificial Sequence
<220>
   <223> Description of Artificial Sequence:probe
<400> 16
   caggaccggc ccgaccgaga caatgaccta cggttggccc agcacatcac ctat 54
<210> 17
   <211> 52
   <212> DNA
   <213> Artificial Sequence
<220>
   <223> Description of Artificial Sequence:probe
<400> 17
   gtgcgctgtg ctaggtcagc accacaacta ccagaactgg agggtgtacg ac 52
<210> 18
   <211> 52
   <212> DNA
   <213> Artificial Sequence
<220>
   <223> Description of Artificial Sequence:probe
<400> 18
   gaagaagact cgagctgcca gggaaggcga ccgacgagat aatcgccttc gg 52
<210> 19
   <211> 52
   <212> DNA
   <213> Artificial Sequence
<220>
   <223> Description of Artificial Sequence:probe
<400> 19
   cactggccga ggataagccc gtccctgtcc cacattctag ccccactatg cg 52
<210> 20
   <211> 60
   <212> DNA
   <213> Artificial Sequence
<220>
   <223> Description of Artificial Sequence:probe
<400> 20
   actgcagcgt atcaaactaa aaggcaccat tgacgtgtca aagctggtta cggggactgt 60
<210> 21
   <211> 60
   <212> DNA
   <213> Artificial Sequence
<220>
   <223> Description of Artificial Sequence:probe
<400> 21
   taataccact acccgtgaat tatatggcct gacaatatga attaggtgta ctgtactgaa 60
<210> 22
   <211> 60
   <212> DNA
   <213> Artificial Sequence
<220>
   <223> Description of Artificial Sequence:probe
<400> 22
   tagtcttccc cagtcctcat tcccagctgc ctcttcctac tgcttccgtc tatcaaaaag 60
<210> 23
   <211> 54
   <212> DNA
   <213> Artificial Sequence
<220>
   <223> Description of Artificial Sequence:probe
<400> 23
   tggccacatg ggaccaagcg gaaccagaac aaggtcccga caggggtaga cgtt 54
<210> 24
   <211> 60
   <212> DNA
   <213> Artificial Sequence
<220>
   <223> Description of Artificial Sequence:probe
<400> 24
   agtcaaagtg cgagtctaca cccatggcat tttcaccatc tctacggcat ctatggtgga 60
<210> 25
   <211> 60
   <212> DNA
   <213> Artificial Sequence
<220>
   <223> Description of Artificial Sequence:probe
<400> 25
   gtcttagaac ctcaactgac atatagcatt gggcacactc cagcagacgc ccgaattcaa 60
<210> 26
   <211> 52
   <212> DNA
   <213> Artificial Sequence
<220>
   <223> Description of Artificial Sequence:probe
<400> 26
   tgggcccaag gctcatgcac acgctaccta ttgtggcacg gagagtaagg ac 52
<210> 27
   <211> 60
   <212> DNA
   <213> Artificial Sequence
<220>
   <223> Description of Artificial Sequence:probe
<400> 27
   ccaccacgct tggccgggat agtatatttt tatagcactt cccctactga ttgctgcctt 60
<210> 28
   <211> 60
   <212> DNA
   <213> Artificial Sequence
<220>
   <223> Description of Artificial Sequence:probe
<400> 28
   gaaatattgc ggtaccaagg ttggctgtac caacatcgcc tatccaacct tagtggtgga 60
<210> 29
   <211> 60
   <212> DNA
   <213> Artificial Sequence
<220>
   <223> Description of Artificial Sequence:probe
<400> 29
   atgacgatta ctactattat ggtccacctc atatgccccc tccaacaaga ggtcgagggc 60
<210> 30
   <211> 60
   <212> DNA
   <213> Artificial Sequence
<220>
   <223> Description of Artificial Sequence:probe
<400> 30
   tatgtctgtg tatgtatagg ggaaagaagg gatccctaac tgttccctta tctgttttct 60
<210> 31
   <211> 60
   <212> DNA
   <213> Artificial Sequence
<220>
   <223> Description of Artificial Sequence:probe
<400> 31
   ctctgcaagc tatcgggatc gtagtgaaac cgatagagct ggttatgcta atggcagtgg 60
<210> 32
   <211> 52
   <212> DNA
   <213> Artificial Sequence
<220>
   <223> Description of Artificial Sequence:probe
<400> 32
   gccggcggca tcgagactat cgctaatgag tatagcgacc gctgcacgcc gg 52
<210> 33
   <211> 52
   <212> DNA
   <213> Artificial Sequence
<220>
   <223> Description of Artificial Sequence:probe
<400> 33
   aaaggcctat cggagctatg tgctgcaccg ctccatggca ctctctaaca cc 52
<210> 34
   <211> 56
   <212> DNA
   <213> Artificial Sequence
<220>
   <223> Description of Artificial Sequence:probe
<400> 34
   aagaacacgc tgtatctgca aatgcacagg ctgagagccg aggacacggc cgtata 56
<210> 35
   <211> 60
   <212> DNA
   <213> Artificial Sequence
<220>
   <223> Description of Artificial Sequence:probe
<400> 35
   gcccggcgat attgtcatta ttgatcagtt cattgacagg actatttgcc acgacatttc 60
<210> 36
   <211> 53
   <212> DNA
   <213> Artificial Sequence
<220>
   <223> Description of Artificial Sequence:probe
<400> 36
   ccctcacatc gcccctcggc tacaacgctt ctctctcgag gcatcgtagc tcg 53
<210> 37
   <211> 56
   <212> DNA
   <213> Artificial Sequence
<220>
   <223> Description of Artificial Sequence:probe
<400> 37
   ggaggactac gacagcttcc ttatgtacag cgatgacgtt ctacgctctc catcgg 56
<210> 38
   <211> 60
   <212> DNA
   <213> Artificial Sequence
<220>
   <223> Description of Artificial Sequence:probe
<400> 38
   ctgcctgacc tttaaaaggc catcgatatt ctttgctcct tgccatagga cttgtattgt 60
<210> 39
   <211> 60
   <212> DNA
   <213> Artificial Sequence
<220>
   <223> Description of Artificial Sequence:probe
<400> 39
   cctttgttaa cactccttac caagtccaca cgactgacga tgacacggaa tgcagtctgg 60
<210> 40
   <211> 60
   <212> DNA
   <213> Artificial Sequence
<220>
   <223> Description of Artificial Sequence:probe
<400> 40
   cagcaccgcc gaagggctct ccttgtcgct cataaagtcc gagtgcggcg atcttcaaga 60
<210> 41
   <211> 60
   <212> DNA
   <213> Artificial Sequence
<220>
   <223> Description of Artificial Sequence:probe
<400> 41
   ggaagtttac gcctgatagc caaacaggga aaaagttaat ggcgaagtgt cgaatgctta 60
<210> 42
   <211> 60
   <212> DNA
   <213> Artificial Sequence
<220>
   <223> Description of Artificial Sequence:probe
<400> 42
   aaaggccgga tgctaggtga tgtgctaatg gatcagaacg tattgcctgg agtagggaac 60
<210> 43
   <211> 56
   <212> DNA
   <213> Artificial Sequence
<220>
   <223> Description of Artificial Sequence:probe
<400> 43
   agcccagttg tagtaggtgc cagtcagtca aggcaggggc cctctctccg tcaata 56
<210> 44
   <211> 54
   <212> DNA
   <213> Artificial Sequence
<220>
   <223> Description of Artificial Sequence:probe
<400> 44
   catgttggcc aagctagtct cgaactactg acttcgggtg atctgccctc ctcg 54
<210> 45
   <211> 53
   <212> DNA
   <213> Artificial Sequence
<220>
   <223> Description of Artificial Sequence:probe
<400> 45
   tcgcccgtca cgcaccgcac gttcgtgggg aacctggcgc taaaccattc gta 53
<210> 46
   <211> 54
   <212> DNA
   <213> Artificial Sequence
<220>
   <223> Description of Artificial Sequence:probe
<400> 46
   ctcaggatac gaagacattt gacgttgcgc tgtccgagga gctccatgcg gcac 54
<210> 47
   <211> 60
   <212> DNA
   <213> Artificial Sequence
<220>
   <223> Description of Artificial Sequence:probe
<400> 47
   gctgatccac cgatacgtgg agctcttaga caaatacttt ggcagtgtgt gcgagctgga 60
<210> 48
   <211> 60
   <212> DNA
   <213> Artificial Sequence
<220>
   <223> Description of Artificial Sequence:probe
<400> 48
   ctctcaaacc aactcgtact gtcgaggtgg attctaaagc agcagaaatt ggtaagaaag 60
(210) 49
   <211> 60
   <212> DNA
   (213) Artificial Sequence
<220>
   <223> Description of Artificial Sequence:probe
(400) 49
   ctcagaaacg gacgacgcaa gaagtgcaag cgacttctag aattcagaac cgaaagtgga 60
<210> 50
   <211> 60
   <212> DNA
   <213> Artificial Sequence
<220>
   <223> Description of Artificial Sequence:probe
(400) 50
   aactggtcct gcaattacta tgaagtcaaa aattaaacta gactatgtct ccaactcagt 60
(210) 51
   (211) 21
   <212> DNA
   <213> Artificial Sequence
<220>
   <223> Description of Artificial Sequence:primer
<400> 51
   gagaagcaca gcatttctga g 21
<210> 52
   (211) 21
   <212> DNA
   (213) Artificial Sequence
<220>
   <223> Description of Artificial Sequence:primer
<400> 52
   tgctctttaa agccttaggc c 21
<210> 53
   <211> 20
   <212> DNA
   <213> Artificial Sequence
<220>
   <223> Description of Artificial Sequence:primer
(400) 53
   aatcctggct actgtgtgag 20
<210> 54
   (211) 19
   <212> DNA
   <213> Artificial Sequence
<220>
   <223> Description of Artificial Sequence:primer
<400> 54
   tccaaagact ggggtaggt 19
<210> 55
   <211> 20
   <212> DNA
   <213> Artificial Sequence
<220>
   <223> Description of Artificial Sequence:primer
<400> 55
   ctactaccgg gcttatgatg 20
<210> 56
   <211> 20
   <212> DNA
   <213> Artificial Sequence
<220>
   <223> Description of Artificial Sequence:primer
<400> 56
   ggaggacttg gtggttttgt 20
<210> 57
   <211> 21
   <212> DNA
   <213> Artificial Sequence
<220>
   <223> Description of Artificial Sequence:primer
<400> 57
   cacgaatatc aggctagaga c 21
<210> 58
   <211> 22
   <212> DNA
   <213> Artificial Sequence
<220>
   <223> Description of Artificial Sequence:primer
<400> 58
   cacatttaca atggctttgg ag 22
<210> 59
   <211> 18
   <212> DNA
   <213> Artificial Sequence
<220>
   <223> Description of Artificial Sequence:primer
<400> 59
   ggctccagtg aaacttgg 18
<210> 60
   <211> 19
   <212> DNA
   <213> Artificial Sequence
<220>
   <223> Description of Artificial Sequence:primer
<400> 60
   caaggtaacg tgagcactt 19
<210> 61
   <211> 20
   <212> DNA
   <213> Artificial Sequence
<220>
   <223> Description of Artificial Sequence:primer
<400> 61
   acccattacg gctactcctc 20
<210> 62
   <211> 21
   <212> DNA
   <213> Artificial Sequence
<220>
   <223> Description of Artificial Sequence:primer
<400> 62
   aaggggtgtt gcttttattg c 21
<210> 63
   <211> 17
   <212> DNA
   <213> Artificial Sequence
<220>
   <223> Description of Artificial Sequence:primer
<400> 63
   cagccatgag gaccatc 17
<210> 64
   <211> 21
   <212> DNA
   <213> Artificial Sequence
<220>
   <223> Description of Artificial Sequence:primer
<400> 64
   tagaaagaca caaggtacac a 21
<210> 65
   <211> 20
   <212> DNA
   <213> Artificial Sequence
<220>
   <223> Description of Artificial Sequence primer
<400> 65
   gagaaaggaa ccgagcttgt 20
<210> 66
   <211> 20
   <212> DNA
   <213> Artificial Sequence
<220>
   <223> Description of Artificial Sequence:primer
<400> 66
   tggtagtaag ggcaagctga 20
<210> 67
   <211> 20
   <212> DNA
   <213> Artificial Sequence
<220>
   <223> Description of Artificial Sequence:primer
<400> 67
   tggcctacct ataactggaa 20
<210> 68
   <211> 20
   <212> DNA
   <213> Artificial Sequence
<220>
   <223> Description of Artificial Sequence:primer
<400> 68
   aatggatgtt ctgcctgaag 20
(210) 69
   <211> 20
   <212> DNA
   <213> Artificial Sequence
<220>
   <223> Description of Artificial Sequence:primer
(400) 69
   gaaggccacc tgaacctaac 20
(210) 70
   (211) 20
   <212> DNA
   <213> Artificial Sequence
<220>
   <223> Description of Artificial Sequence:primer
<400> 70
   ccatctgaag actccgacag 20
<210> 71
   <211> 21
   <212> DNA
   <213> Artificial Sequence
<220>
   <223> Description of Artificial Sequence:primer
<400> 71
   gatcttcaag tcctcatcac c 21
(210) 72
   <211> 20
   <212> DNA
   <213> Artificial Sequence
<220>
   <223> Description of Artificial Sequence:primer
<400> 72
   accagcttta actcaccttc 20
(210) 73
   <211> 21
   <212> DNA
   (213) Artificial Sequence
<220>
   (223) Description of Artificial Sequence:primer
<400> 73
   gtacgtccag aaaaggctat g 21
<210> 74
   <211> 19
   <212> DNA
   <213> Artificial Sequence
<220>
   <223> Description of Artificial Sequence:primer
<400> 74
   ggacttaggg tcatcggaa 19
<210> 75
   <211> 18
   <212> DNA
   <213> Artificial Sequence
<220>
   <223> Description of Artificial Sequence:primer
<400> 75
   aggtgacatc cacgaact 18
<210> 76
   <211> 22
   <212> DNA
   <213> Artificial Sequence
<220>
   <223> Description of Artificial Sequence:primer
<400> 76
   gtaggaaagt agagctttac cc 22
<210> 77
   <211> 19
   <212> DNA
   <213> Artificial Sequence
<220>
   <223> Description of Artificial Sequence:primer
<400> 77
   cagttcgtcc ggcttcctc 19
<210> 78
   <211> 24
   <212> DNA
   <213> Artificial Sequence
<220>
   <223> Description of Artificial Sequence:primer
<400> 78
   atgaagatga gtccatgttc tcgt 24
<210> 79
   <211> 17
   <212> DNA
   <213> Artificial Sequence
<220>
   <223> Description of Artificial Sequence:primer
<400> 79
   actgcaccca tgagact 17
<210> 80
   <211> 20
   <212> DNA
   <213> Artificial Sequence
<220>
   <223> Description of Artificial Sequence:primer
<400> 80
   gttcatggtg atgcctacaa 20
<210> 81
   <211> 20
   <212> DNA
   <213> Artificial Sequence
<220>
   <223> Description of Artificial Sequence:primer
<400> 81
   gtggagaact gaccttagag 20
<210> 82
   <211> 21
   <212> DNA
   <213> Artificial Sequence
<220>
   <223> Description of Artificial Sequence:primer
<400> 82
   tcctttgaca tctccattag c 21
<210> 83
   <211> 19
   <212> DNA
   <213> Artificial Sequence
<220>
   <223> Description of Artificial Sequence:primer
<400> 83
   ctcctcctgt tcctatgtg 19
<210> 84
   <211> 18
   <212> DNA
   <213> Artificial Sequence
<220>
   <223> Description of Artificial Sequence:primer
<400> 84
   gcaggagaag catcgatg 18
<210> 85
   <211> 20
   <212> DNA
   <213> Artificial Sequence
<220>
   <223> Description of Artificial Sequence:primer
<400> 85
   agagtgcgag aacaacgaat 20
<210> 86
   <211> 21
   <212> DNA
   <213> Artificial Sequence
<220>
   <223> Description of Artificial Sequence:primer
<400> 86
   actaaaggtc aggaaaggga a 21
<210> 87
   <211> 20
   <212> DNA
   <213> Artificial Sequence
<220>
   <223> Description of Artificial Sequence:primer
<400> 87
   cttcacctgc agagtccttt 20
<210> 88
   <211> 20
   <212> DNA
   <213> Artificial Sequence
<220>
   <223> Description of Artificial Sequence:primer
<400> 88
   gatagagggt catgcagtgg 20
<210> 89
   <211> 20
   <212> DNA
   <213> Artificial Sequence
<220>
   <223> Description of Artificial Sequence:primer
<400> 89
   caactcctca caacccttcc 20
<210> 90
   <211> 20
   <212> DNA
   <213> Artificial Sequence
<220>
   <223> Description of Artificial Sequence:primer
<400> 90
   ttcttggtga ggtatttggc 20
<210> 91
   <211> 19
   <212> DNA
   <213> Artificial Sequence
<220>
   <223> Description of Artificial Sequence:primer
<400> 91
   gaagaaacgc ctcaaaagc 19
<210> 92
   <211> 24
   <212> DNA
   <213> Artificial Sequence
<220>
   <223> Description of Artificial Sequence:primer
<400> 92
   ttttctttaa tacagttgcc atct 24
<210> 93
   <211> 20
   <212> DNA
   <213> Artificial Sequence
<220>
   <223> Description of Artificial Sequence:primer
<400> 93
   caagctgtca ctccccatac 20
<210> 94
   <211> 20
   <212> DNA
   <213> Artificial Sequence
<220>
   <223> Description of Artificial Sequence:primer
<400> 94
   gggaggtcag gctttagaga 20
<210> 95
   <211> 19
   <212> DNA
   <213> Artificial Sequence
<220>
   <223> Description of Artificial Sequence:primer
<400> 95
   tggtttctgc catccggaa 19
<210> 96
   <211> 22
   <212> DNA
   <213> Artificial Sequence
<220>
   <223> Description of Artificial Sequence:primer
<400> 96
   cttgatcctc tctagcgcgt aa 22
<210> 97
   <211> 18
   <212> DNA
   <213> Artificial Sequence
<220>
   <223> Description of Artificial Sequence:primer
<400> 97
   caatactttc cccggcat 18
<210> 98
   <211> 18
   <212> DNA
   <213> Artificial Sequence
<220>
   <223> Description of Artificial Sequence:primer
<400> 98
   cctaactgcc agaccaag 18
<210> 99
   (211) 20
   (212) DNA
   <213> Artificial Sequence
<220>
   <223> Description of Artificial Sequence:primer
<400> 99
   cgcagacctt gtgatattcc 20
<210> 100
   <211> 23
   <212> DNA
   <213> Artificial Sequence
<220>
   <223> Description of Artificial Sequence:primer
<400> 100
   cgattccctc tcatttattc ctt 23
<210> 101
   <211> 20
   <212> DNA
   <213> Artificial Sequence
<220>
   <223> Description of Artificial Sequence:primer
<400> 101
   gaggatgatg agctgctaca 20
<210> 102
   <211> 22
   <212> DNA
   <213> Artificial Sequence
<220>
   <223> Description of Artificial Sequence:primer
<400> 102
   ctacacactt ttattggagg gg 22
<210> 103
   <211> 23
   <212> DNA
   <213> Artificial Sequence
<220>
   <223> Description of Artificial Sequence:primer
<400> 103
   aactcattta cggataggac ttt 23
<210> 104
   <211> 20
   <212> DNA
   <213> Artificial Sequence
<220>
   <223> Description of Artificial Sequence:primer
<400> 104
   ttcctgtcct attggtacca 20
<210> 105
   <211> 20
   <212> DNA
   <213> Artificial Sequence
<220>
   <223> Description of Artificial Sequence:primer
<400> 105
   aaatgctaca ctccaagggc 20
<210> 106
   <211> 21
   <212> DNA
   <213> Artificial Sequence
<220>
   <223> Description of Artificial Sequence:primer
<400> 106
   cagaaaatag caagcaggaa g 21
<210> 107
   <211> 22
   <212> DNA
   <213> Artificial Sequence
<220>
   <223> Description of Artificial Sequence:primer
<400> 107
   aatgggctga tcctatagaa ga 22
<210> 108
   <211> 20
   <212> DNA
   <213> Artificial Sequence
<220>
   <223> Description of Artificial Sequence:primer
<400> 108
   ctctttgttg ttgggcacct 20
<210> 109
   <211> 21
   <212> DNA
   <213> Artificial Sequence
<220>
   <223> Description of Artificial Sequence:primer
<400> 109
   acctcatctc caaactgctc a 21
<210> 110
   <211> 25
   <212> DNA
   <213> Artificial Sequence
<220>
   <223> Description of Artificial Sequence:primer
<400> 110
   aaaaagcttc agcctttatt aaaca 25
<210> 111
   <211> 21
   <212> DNA
   <213> Artificial Sequence
<220>
   <223> Description of Artificial Sequence:primer
<400> 111
   ctcagaggat tatgtgcacc g 21
<210> 112
   <211> 20
   <212> DNA
   <213> Artificial Sequence
<220>
   <223> Description of Artificial Sequence:primer
<400> 112
   aggaggagga gggtattggt 20
<210> 113
   <211> 22
   <212> DNA
   <213> Artificial Sequence
<220>
   <223> Description of Artificial Sequence:primer
<400> 113
   cagtacccac tggaaggact ta 22
<210> 114
   <211> 22
   <212> DNA
   <213> Artificial Sequence
<220>
   <223> Description of Artificial Sequence:primer
<400> 114
   tctccttcag tttggacaaa ag 22
<210> 115
   <211> 20
   <212> DNA
   <213> Artificial Sequence
<220>
   <223> Description of Artificial Sequence:primer
<400> 115
   agaacttcaa tgtggccacg 20
<210> 116
   <211> 20
   <212> DNA
   <213> Artificial Sequence
<220>
   <223> Description of Artificial Sequence:primer
<400> 116
   catactggtg gcttcatctt 20
<210> 117
   <211> 21
   <212> DNA
   <213> Artificial Sequence
<220>
   <223> Description of Artificial Sequence:primer
<400> 117
   gtgggtctca gctattagtg g 21
<210> 118
   <211> 23
   <212> DNA
   <213> Artificial Sequence
<220>
   <223> Description of Artificial Sequence:primer
<400> 118
   ggatttcgca cagtaatata egg 23
<210> 119
   <211> 20
   <212> DNA
   <213> Artificial Sequence
<220>
   <223> Description of Artificial Sequence:primer
<400> 119
   ctcgcttggt tcccttagtc 20
<210> 120
   <211> 20
   <212> DNA
   <213> Artificial Sequence
<220>
   <223> Description of Artificial Sequence:primer
<400> 120
   atccctgcag gaaaaatcat 20
<210> 121
   <211> 20
   <212> DNA
   <213> Artificial Sequence
<220>
   <223> Description of Artificial Sequence:primer
<400> 121
   atgtcaaggg tgcatcagtc 20
<210> 122
   <211> 20
   <212> DNA
   <213> Artificial Sequence
<220>
   <223> Description of Artificial Sequence:primer
<400> 122
   gaacccttct gaacctggtc 20
<210> 123
   <211> 20
   <212> DNA
   <213> Artificial Sequence
<220>
   <223> Description of Artificial Sequence:primer
<400> 123
   tctggccttc aatgtcgtct 20
<210> 124
   <211> 20
   **(212)** DNA
   <213> Artificial Sequence
<220>
   <223> Description of Artificial Sequence:primer
<400> 124
   tgtggtcgag tgtgagtgtt 20
<210> 125
   <211> 20
   <212> DNA
   <213> Artificial Sequence
<220>
   <223> Description of Artificial Sequence:primer
<400> 125
   atgtccatgt gaacgctact 20
<210> 126
   <211> 20
   <212> DNA
   <213> Artificial Sequence
<220>
   <223> Description of Artificial Sequence:primer
<400> 126
   ccaagcctct ggttctgatg 20
<210> 127
   <211> 20
   <212> DNA
   <213> Artificial Sequence
<220>
   <223> Description of Artificial Sequence:primer
<400> 127
   caagagtggt ggatcggttc 20
<210> 128
   <211> 20
   <212> DNA
   <213> Artificial Sequence
<220>
   <223> Description of Artificial Sequence:primer
(400) 128
   tcaacaagtg gagaaggcaa 20
<210> 129
   <211> 20
   <212> DNA
   <213> Artificial Sequence
<220>
   <223> Description of Artificial Sequence:primer
<400> 129
   tgatggccta tccatttcag 20
<210> 130
   <211> 20
   <212> DNA
   <213> Artificial Sequence
<220>
   <223> Description of Artificial Sequence:primer
<400> 130
   aacatctttg cctgcgataa 20
<210> 131
   <211> 20
   <212> DNA
   <213> Artificial Sequence
<220>
   <223> Description of Artificial Sequence:primer
<400> 131
   aagcaacaac agcaggagtc 20
<210> 132
   <211> 20
   <212> DNA
   <213> Artificial Sequence
<220>
   <223> Description of Artificial Sequence:primer
<400> 132
   tgtgaaatcc agacccagac 20
<210> 133
   <211> 20
   <212> DNA
   <213> Artificial Sequence
<220>
   <223> Description of Artificial Sequence:primer
<400> 133
   atttcggaat gaaaggcttc 20
<210> 134
   <211> 20
   <212> DNA
   <213> Artificial Sequence
<220>
   <223> Description of Artificial Sequence:primer
<400> 134
   ccctgctaga tgtccaactg 20
<210> 135
   <211> 20
   <212> DNA
   <213> Artificial Sequence
<220>
   <223> Description of Artificial Sequence:primer
<400> 135
   gcttataccc tgttcccaaa 20
<210> 136
   <211> 23
   <212> DNA
   <213> Artificial Sequence
<220>
   <223> Description of Artificial Sequence:primer
<400> 136
   acacagctcc atcataattt cat 23
<210> 137
   <211> 24
   <212> DNA
   <213> Artificial Sequence
<220>
   <223> Description of Artificial Sequence:primer
<400> 137
   gctcagtgct agtctgttgt gtag 24
<210> 138
   <211> 19
   <212> DNA
   <213> Artificial Sequence
<220>
   <223> Description of Artificial Sequence:primer
<400> 138
   ccctggcctc aagtgatcc 19
<210> 139
   <211> 19
   <212> DNA
   <213> Artificial Sequence
<220>
   <223> Description of Artificial Sequence:primer
<400> 139
   gaattcacca agcgttgga 19
<210> 140
   <211> 22
   <212> DNA
   <213> Artificial Sequence
<220>
   <223> Description of Artificial Sequence:primer
<400>
   gctgactttc aatagatcgc ag 22
<210> 141
   <211> 20
   <212> DNA
   <213> Artificial Sequence
<220>
   <223> Description of Artificial Sequence:primer
<400> 141
   tgttcctctg gaactggaga 20
<210> 142
   <211> 20
   <212> DNA
   <213> Artificial Sequence
<220>
   <223> Description of Artificial Sequence:primer
<400> 142
   ccctcctttt gctcaagaat 20
<210> 143
   <211> 19
   <212> DNA
   <213> Artificial Sequence
<220>
   <223> Description of Artificial Sequence:primer
<400> 143
   gaaagcccaa gatgtgcag 19
<210> 144
   <211> 19
   <212> DNA
   <213> Artificial Sequence
<220>
   <223> Description of Artificial Sequence:primer
<400> 144
   gagtcccacc aagaacagg 19
<210> 145
   <211> 23
   <212> DNA
   <213> Artificial Sequence
<220>
   <223> Description of Artificial Sequence:primer
<400> 145
   agagtaaact ttgggccata tga 23
<210> 146
   <211> 24
   <212> DNA
   <213> Artificial Sequence
<220>
   <223> Description of Artificial Sequence:primer
<400> 146
   accttttttg ctatctttct ctgt 24
<210> 147
   <211> 20
   <212> DNA
   <213> Artificial Sequence
<220>
   <223> Description of Artificial Sequence:primer
<400> 147
   cttgaatcat gggtgacgtt 20
<210> 148
   <211> 20
   <212> DNA
   <213> Artificial Sequence
<220>
   <223> Description of Artificial Sequence:primer
<400> 148
   gtttggtgcg attatgtggt 20
<210> 149
   *<211>* 23
   <212> DNA
   <213> Artificial Sequence
<220>
   <223> Description of Artificial Sequence:primer
<400> 149
   ctcaggattc aagaaatgga agg 23
<210> 150
   <211> 22
   <212> DNA
   <213> Artificial Sequence
<220>
   <223> Description of Artificial Sequence:primer
<400> 150
   gtgaaggtac tgaagatcag cg 22
<210> 151
   <211> 60
   <212> DNA
   <213> Artificial Sequence
<220>
   <223> Description of Artificial Sequence:probe
<400> 151
   ccatagtcaa tgatggttta ataggtaaac caaaccctat aaacctgacc tcctttatgg 60
<210> 152
   <211> 60
   <212> DNA
   <213> Artificial Sequence
<220>
   <223> Description of Artificial Sequence:probe
<400> 152
   ccaactgtca accttgacaa attgtggact ttggtcagtg aacagacacg ggtgaatgct 60
<210> 153
   <211> 60
   <212> DNA
   <213> Artificial Sequence
<220>
   <223> Description of Artificial Sequence:probe
<400> 153
   gagtgtaagg cgtacggtga gaacattggg tacagtgaga aagaccgttt tcagggacgt 60
<210> 154
   <211> 60
   <212> DNA
   <213> Artificial Sequence
<220>
   <223> Description of Artificial Sequence:probe
<400> 154
   ccaccccact cttaatcagt ggtggaagaa cggtctcaga actgtttgtt tcaattggcc 60
<210> 155
   <211> 56
   <212> DNA
   <213> Artificial Sequence
<220>
   <223> Description of Artificial Sequence:probe
<400> 155
   ctctgatcgt aggaattgag gagtgtcccg ccttgtggct gagaactgga cagtgg 56
<210> 156
   <211> 55
   <212> DNA
   <213> Artificial Sequence
<220>
   <223> Description of Artificial Sequence:probe
<400> 156
   tcatccgccg agactatctg cactacatcc gcaagtacaa ccgcttcgag aagcg 55
<210> 157
   <211> 60
   <212> DNA
   <213> Artificial Sequence
<220>
   <223> Description of Artificial Sequence:probe
<400> 157
   acatccagca gtggtcattc gacaacgaaa gtcataccgt agaaaagatg gcgtgtttct 60
<210> 158
   <211> 22
   <212> DNA
   <213> Artificial Sequence
<220>
   <223> Description of Artificial Sequence:primer
<400> 158
   aattagcagt ttagaatgga gg 22
<210> 159
   <211> 20
   <212> DNA
   <213> Artificial Sequence
<220>
   <223> Description of Artificial Sequence:primer
<400> 159
   tgggctgcca acatgccatc 20
<210> 160
   <211> 22
   <212> DNA
   <213> Artificial Sequence
<220>
   <223> Description of Artificial Sequence:primer
<400> 160
   ggcaagcgag atgaagataa gg 22
<210> 161
   <211> 21
   <212> DNA
   <213> Artificial Sequence
<220>
   <223> Description of Artificial Sequence:primer
<400> 161
   agactatcca cctttgggtc g 21
<210> 162
   <211> 22
   <212> DNA
   <213> Artificial Sequence
<220>
   <223> Description of Artificial Sequence:primer
<400> 162
   ttgagcgcac ctaaccactg gt 22
<210> 163
   <211> 22
   <212> DNA
   <213> Artificial Sequence
<220>
   <223> Description of Artificial Sequence:primer
<400> 163
   acattcagac tgagcgtgcc ta 22
<210> 164
   <211> 22
   <212> DNA
   <213> Artificial Sequence
<220>
   <223> Description of Artificial Sequence:primer
<400> 164
   ttcaagatgt cgaagcgagg ac 22
<210> 165
   <211> 21
   <212> DNA
   <213> Artificial Sequence
<220>
   <223> Description of Artificial Sequence:primer
<400> 165
   ctgtatccaa ttctgtactg c 21
<210> 166
   <211> 20
   <212> DNA
   <213> Artificial Sequence
<220>
   <223> Description of Artificial Sequence:primer
<400> 166
   tgtagtagcc cgatcgcacc 20
<210> 167
   <211> 20
   <212> DNA
   <213> Artificial Sequence
<220>
   <223> Description of Artificial Sequence:primer
<400> 167
   aggtcccata cgtatgacag 20
<210> 168
   <211> 25
   <212> DNA
   <213> Artificial Sequence
<220>
   <223> Description of Artificial Sequence:primer
<400> 168
   gatgcattgt tatcattaac cagtc 25
<210> 169
   <211> 23
   <212> DNA
   <213> Artificial Sequence
<220>
   <223> Description of Artificial Sequence:primer
<400> 169
   gagaggaaac atggtcacac cca 23
<210> 170
   <211> 21
   <212> DNA
   <213> Artificial Sequence
<220>
   <223> Description of Artificial Sequence:primer
<400> 170
   gatctggacg tccctgaagc a 21
<210> 171
   <211> 25
   <212> DNA
   <213> Artificial Sequence
<220>
   <223> Description of Artificial Sequence:primer
<400> 171
   tgtaatggca gaacctttca tctcg 25

## Description

### BACKGROUND OF THE INVENTION

### Field of the Invention

The present invention relates to an examination method for early colon cancer. Stated more in detail, the present invention relates to a method for screening colon cancer cells in which the expression amount of a specific set of genes in a sample (blood, stool, and the like) is used as an indicator. The present invention also relates to primers, probes, and immobilized samples for this method.

### Description of the Related Art

The most frequent cause of cancer death in Japanese is stomach cancer. However, in recent years, the number of cases of stomach cancer has been decreasing, and instead, colon cancer has shown a dramatic increase. The ratio of colon cancer among all cancer deaths has been increasing annually from 1955. It is said that in the 21st century, the number of colon cancer deaths will surpass that of stomach cancer deaths to become the top.

On the other hand, colon cancer advances relatively slowly. Even in advanced cancer cases, as long as a complete curative resection is conducted, prognosis is relatively good. Five year survival rates, for example, for Dukes' A, Dukes' B and Dukes' C is 95%, 80% and 50-60%, respectively. However, there are an ignorable number of cases where no or little subjective symptom appears until a fairly advanced stage and at the time of definitive diagnosis, the cancer has already metastasized or become invasive and resection is no longer possible. Therefore, early detection is strongly needed (see cancer statistics by the National Cancer Center, Tokyo).

Currently, the main method that is used for screening of colon cancer is a fecal occult blood test. In a fecal occult blood test, hemoglobin in blood is chemically measured to detect bleeding from the surface of colonic lumen which cannot be seen by the naked eye can be detected. This method is extremely sensitive, and even a small amount of blood in the stool can be detected. However, while the chemical occult blood test has good sensitivity, this test is not specific to human hemoglobin. False positives are seen when there is a reaction with meat or green vegetables that is eaten or due to medications. Prior to examination, strict dietary restriction is required.

In recent years, an immunological fecal occult blood reaction method has been developed. This method specifically detects human hemoglobin in stool using an antibody and is currently used in actual examination. While the immunologic fecal occult blood reaction specifically detects hemoglobin in stool, hemoglobin easily breaks down in stool, and as a result, there is the problem that, with this immunologic method, hemoglobin that has been broken down cannot be measured.

In addition, the positivity rate for advanced cancers is 90% with this method, but for all stages, which combines early cancers and advanced cancers, the positivity rate is only 50% (Launoy G et al., Int. J. Cancer 1997, 73:220-224). In other words, there is the possibility that one out of two colon cancer cases will be missed. In addition, because this is a detection method which confirms the presence of bleeding, this test is positive for reasons other than cancers, such as hemorrhoids. The probability of having colon cancer among people with positive reaction (positive predictive value) is only approximately 1-2% (Mandel JS et al., N. Engl. J. Med., 2000, 343 (22): 1603-1607). Furthermore, false positive rate (the probability of the test being positive in healthy individuals) is between 5-10%. Further improvement is desired.

On the other hand, diagnosis methods using tumor markers have been proposed. Tumor markers for colon cancer include carcinoembryonic antigen (CEA), CA19-9, NCC-ST-439, STN, and the like. These are used for determining treatment effectiveness and for monitoring of recurrence (Okura, Hisanao et al, Tumor markers for colon cancer, CRC 1(4) 42-47, 1992). There has also been a research into methods which target mutations of DNA (K-RAS, P53, APC, and the like) in stool. However, there are difficulties in implementing these methods targeting mutations in DNA in stool, and these methods are still only in their research stage.

In those methods relying on tumor markers, the tumor marker positivity rates, even with Dukes' C for which curative resection is possible, are only 36%, 30%, 35% and 21% for CEA, CA19-9, NCC-ST-439 and STN, respectively. Thus, it cannot be said that these tumor markers are adequate for early colon cancers (Okura, Hisanao et al., Tumor markers for colon cancer, CRC 1(4), 42-47, 1992).

### SUMMARY OF THE INVENTION

The object of the present invention is to provide an early diagnosis method for colon cancer in which colon cancer patients are detected with high precision as compared to the prior method.

This object is achieved by the screening method according to claim 1. Claims 2 to 6 relate to further developments. In these methods, use can be made of a primer as claimed in claim 7, of a probe as claimed in claim 8, of a solid-phase carrier as claimed in claim 9, and of a detection kit as claimed in claim 10.

The present inventors have conducted intensive study in order to solve the above problems. The present inventors have then identified 57 types of genes which are closely associated with colon cancer cells. The present inventors have discovered further that, by measuring expression levels of those genes, colon cancer patients can be detected with high precision.

As a probe for determining the expression levels of those genes, partial base sequences specific thereto have been identified. In addition, primers which can specifically amplify very small amounts of mRNAs of those genes in a sample have been designed.

In addition, a solid phase carrier of those probes has been provided, and by reacting it with labeled cDNAs in a multiplex RT-PCR (where a plurality of cDNAs are amplified by PCR in one tube), a method for simultaneously measuring the expression levels of a plurality of genes has been developed.

The present invention as claimed provides a method for screening of colon cancer cells in a sample by analyzing an amount of expression of at least 2 or more genes, or products thereof, selected from the group of genes listed in Table 26.

Of the group of genes listed in Table 1, the genes listed in Tables 26, 28 and 30 are genes which particularly differentiate colon cancer from hemorrhoids. Even if blood is contained in a sample, they are suitably used for screening, or judging the presence or absence of, cancer cells.

In the present invention, the expression amount of a gene is analyzed by measuring an amount of a mRNA in a sample. The expression amount of a gene product, on the other hand, is analyzed by using an antibody against the gene product. As a sample, a stool smear obtained from a subject is used. When a stool smear is used, in order to measure the expression levels of respective genes in colon cancer cells released in the stool, a test sample is prepared in which a buffer is added at room temperature to the naturally excreted stool, and impurities are removed. The cancer cells in the sample are then adsorbed onto a solid phase carrier, and the adsorbed cancer cells are collected. With this procedure, it is possible to recover live colon cancer cells released in the stool efficiently.

The genes listed in Table 28 and Table 30 are genes which are particularly suitable for screening for the presence or absence of small amounts of colon cancer cells released in stool.

With the above colon cancer cell screening, examination and diagnosis of colon cancer, particularly of early colon cancer, can be made easily. The present invention also provides an examination method for colon cancer in vitro.

Furthermore, for the present invention there are provided a primer for amplifying specifically any one of the genes listed in Table 26, a probe specifically hybridizing with any one of the genes listed in Table 26 for detection of the gene, and an immobilized sample in which the probe is immobilized on a solid-phase carrier. These primers, probes, and/or immobilized samples can be used in examination for colon cancer as a gene detection kit for the genes listed in Table 26.

There are further provided a gene set (gene marker set) for colon cancer testing of at least two or more genes selected from the genes listed in Table 26, and a gene set for colon cancer testing of at least two or more genes selected from the 7 genes listed in Table 30. Also provided are primers, probes and immobilized samples for analyzing the expression of these genes. According to the present invention, because the expression of genes can be simultaneously analyzed in the sample, early diagnosis of colon cancer is easily carried out.

Further features of the present invention will become apparent from the following description of exemplary embodiments with reference to the attached drawings.

### BRIEF DESCRIPTION OF THE DRAWINGS

FIG. 1 shows results of RT-PCR of RNAs of blood and surgically resected colon cancer tissues for selected genes.
FIG. 2 shows results of RT-PCR of RNAs collected from stool samples.
FIG. 3 shows chip hybridization results of RNAs collected from stool samples.

### DESCRIPTION OF THE EMBODIMENTS

The present invention is described below in detail. Any description is one example of implementing the present invention, and they by no means restricts the present invention.

With the present invention, in order to provide information useful for diagnosing colon cancer, 50 types of genes which were judged to have a high amount of expression in colon cancer tissue but no or extremely low expression in normal tunica mucosa coli were selected through various expression analyses.

With a commercial microarray, the expression profiles for approximately 39000 genes were obtained for early colon cancer tissue, advanced colon cancer tissue, and normal tunica mucosa coli. Using these results and a public database, 50 genes were selected (Table 1). Of these, there were 30 genes whose expression was not detected in colon mucosa or peripheral blood and which were strongly expressed in early colon cancer (Dukes' A, B) and which is still expressed in advanced colon cancer (Dukes' C, D). There were 16 genes which were strongly expressed in advanced colon cancer and whose expression was still detected in early colon cancer. There were 4 genes which were expressed strongly in all stages of cancers (Dukes' A-D).

Furthermore, through RT-PCR, 15 genes which were positive for one or both of early colon cancer (22 cases of mixed samples) and advanced colon cancer (8 cases of mixed samples) and which were confirmed to be negative in peripheral blood was selected (Table 26). These 15 types of genes were negative even with nested PCR (high cycle PCR conducted twice with 2 sets of primers). These are genes which can differentiate between hemorrhoids and colon cancer. In other words, by using expression of these genes as an indictor, screening for presence or absence of cancer cells can be conducted even if blood is contained in the sample.

In addition, with cells obtained from the stool of colon cancer subjects and healthy subjects, analysis with a commercial microarray and RT-PCR were conducted, and 7 genes (Table 30) that can be used to screen for the presence or absence of cancer cells were selected. The genes listed in Table 28 and Table 30 are extremely good for screening for cancer cells in cells obtained from stool.

For the screening method of the present invention, colon cancer screening is conducted using a gene set described in Table 1, Table 26, Table 28, or Table' 30. Screening is conducted by measuring the expression amount of 2 or more genes selected from the gene set. The expression amount of the genes can be measured by measuring the mRNA amount in the sample or it can be detected by using immunostaining or ELISA of the protein which is the gene product.

For the genes listed in Tables 1, 26, 28, and 30, examples of a suitable base sequence for a probe for specifically detecting each gene is indicated by SEQ ID NOs: 1-50 and 151-157. Each probe contains the partial sequence for the base sequence of each gene. However, the probes are made so that non-specific hybridization with partial sequences from other genes is prevented as much as possible. The probes all have a chain length of 50-60 mer base length. With the assumption that there will be simultaneous hybridization with a plurality of probes, the probes are adjusted so that there is not a large variability among the probes in Tm values and the like. However, as long as the desired effect is not lost, looking at the base sequence of the target gene, the base length can be adjusted as suitable. In addition, as long as the specificity to the corresponding gene is not lost, each of the probes can be designed to have a base sequence in which 1 or more bases are deleted, substituted, or added with respect to the base sequences indicated in Table 26.

In addition, with the nucleic acids extracted from cells obtained from the sample (human stool), for example, DNA can be hybridized with the probe as a double stranded DNA. As a result, the sequence for the probe used in such a situation can be the complementary sequence to the base sequence shown in Table 26. With the screening method of the present invention as claimed, two or more of these probes are used as a set. In other words, the present invention as claimed provides a probe set which can detect 2 or more types of genes selected from the genes listed in Table 26. Furthermore, the present invention provides a probe set which can detect 2 or more genes selected from genes listed in Table 30.

The probes of the present invention are highly specific, and they have a strict one-to-one correspondence with the target gene. As a result, there is no cross-hybridization, and a plurality of types of probes can be used simultaneously. The probe of the present invention can be used in the liquid phase or solid phase, but from the standpoint of simultaneous detection of a plurality of genes, preferably, each probe is immobilized on a carrier which is physically separated.

When using in the solid phase, the method for immobilization of the probe is not limited. Known immobilization methods such as adsorption, ionic bonding, covalent bonding and the like can be used as appropriate. In this situation, in order to have a stronger bond, as long as there is no significant loss of hybridization between sample and probe, there can be chemical modification of the probe, and the bonding can take advantage of the modification residue. Examples of such a chemical modification include methods for introducing an amino group to the 5' terminus or methods for introducing a thiol group or methods for modifying with biotin.

The form of the solid-phase carrier is not particularly limited and can be a flat substrate, beads, fibers, and the like. In addition, the material is not limited and can be metal, glass, polymer, or the like.

A suitable example of an immobilized sample includes a DNA microarray in which a 'plurality of genes can be detected simultaneously with high sensitivity. A DNA microarray is a device for detecting nucleic acids in which a plurality of probes are arranged in a dense array on the surface of a flat substrate. Various known methods can be used to create DNA microarrays. In one example, glass is used as the solid-phase carrier. This glass is treated with an amino silane coupling agent which introduces an amino group. After further introducing a maleimido group onto the surface through EMCS or the like, an oligonucleotide probe which has been modified with a thiol group on the 5' terminus reacts with the maleimido group. The probe is brought to the glass surface through a covalent bond.

When the carrier is a flat carrier such as a glass substrate, pipetting and the like is a representative means for supplying the probe onto the surface of the carrier. However, in order to supply a smaller amount of probe solution at high density, liquid supplying methods using ink jet methods such as bubble jet method or piezo method are used.

For the screening method of the present invention, there are two main pre-treatments which are conducted on the sample. These pre-treatments are labeling for detection and amplification to improve sensitivity. However, labeling is not always necessary if, after hybridization with the probe, there is a separate means for detecting hybridization of the probe with the sample. In addition, if the detection target is present in the sample in large quantities, amplification is not always needed.

Because in general there is only a small quantity of sample RNA (submicrogram), an amplification step is usually necessary. For the amplification method, in vitro transcription reaction or RT-PCR reaction is generally used. With amplification by RT-PCR, for the region to be amplified, in other words in the nucleic acid base sequence of each gene, the two primers which surround the region set by the probe must be set accurately. For each gene listed in Table 1, Table 26, Table 28, and Table 30 (Table 26 claimed in the present invention), primers which can specifically amplify these genes were designed. An example of a suitable base sequence for each primer is indicated by SEQ ID NOs: 51-150 and 158-171. As with the probe, with the primer, it is assumed that there will be simultaneous amplification of a plurality of genes. The primers are adjusted so that there are no large variations in Tm values and the like among the primers. However, as long as the desired specificity and amplification rate is not diminished, the base sequence can be added or subtracted. For the addition to or subtraction from the base sequence, one or several bases is added or subtracted from the 5' terminus or 3' terminus or from both.

The labeling of the sample RNA is easily implemented by using a labeled substrate in the amplification step described above. Alternatively, there is a method in which the primer itself is labeled in advance. There is also a method in which, after the amplification step, a labeling substance is chemically or enzymatically bonded to a prescribed functional group of the sample. Labeling methods include known labeling methods such as fluorescent labeling, radiolabeling, enzymatic labeling and the like.

In the PCR reaction, the primers of the present invention have a high specificity with respect to the genes. Several types can be used in combination. A combined primer sets can be used in RT-PCR with the sample RNA as a template.

In addition, by combining these primer sets with an immobilized sample as described above, for example the DNA microarray, this can be used as a. kit to detect specific genes. Of course, even just the primer set diluted in a suitable buffer solution can be used as a gene detection kit.

The genes selected in the present invention have a strong association with colon cancer cells. As a result, by measuring the expression levels of these genes, screening for colon cancer cells can be conducted. In particular, the gene expression profile which is measured using the probe of the present invention can be used for early diagnosis of colon cancer.

With the method of the present invention, the expression levels of each gene are measured with high sensitivity through fluorescent intensity or radiation intensity of the labeled probe. With regard to measurements, the appropriate standardization is conducted for each probe and each sample. By conducting a prognostication which can be compared between samples, a more accurate determination is possible. For example, when there is a difference in the amount of RNA recovered for each sample, by comparing the expression amount of the target gene with the expression amount of genes which have a constant expression amount such as housekeeping genes (for example beta actin), adjustments to the recovery amount are possible. In addition, if the bonding force of the probe with respect to a sample which has a constant concentration is different for each probe, then a calibration graph (calibration curve) can be created using the luminance of an artificially synthesized synthetic sample of known concentration. Referring to this value, the expression levels for target genes contained in the sample can be measured. By making a determination based on the measurement results, a more accurate determination is possible.

The probe, primer, immobilized sample, as well as the gene detection method of the present invention can be used for colon cancer diagnosis as described above. However, even with different objectives or samples, the present disclosure can be used for detecting genes described in Table 1, Table 26, Table 28, and Table 30 (Table 26 for the invention as claimed).

The screening of colon cancer cells is conducted by analysis of genes (mRNA) as described above. In addition, the present invention as claimed can be implemented by analyzing the expression amount of the proteins which are the translation product of the genes in Table 26. The analysis of the expression amount of the proteins which are the gene products is implemented by known methods, such as western blotting method, dot blotting, slot blotting method, ELISA method, and RIA method, using antibody specific to the protein.

### EXAMPLES

Below, we describe the present invention in further detail by showing concrete embodiments as well as reference examples outside the scope of the invention as claimed.

### Example 1

(Selection step 1) Primary selection of marker genes for colon cancer screening.

### (1) Total RNA extraction

Peripheral blood, 6 cases of normal tunica mucosa coli, 6 cases of early colon cancer tissue (Dukes' A, B) and 19 cases of advanced colon cancer tissue (Dukes' C, D) were collected, and total RNA was recovered. Recovery of total RNA was conducted according to the usual methods, and the following method was conducted.

First, each tissue sample was crushed (peripheral blood was used as it is), and ISOGEN from Nippon Gene Co. was added, and this was homogenized. A small amount of chloroform was added. This was centrifuged at 8000 rpm for 15 minutes. The supernatant was collected, and an equal amount of isopropanol to the collection amount was added. This was incubated for 15 minutes or longer at room temperature. This was centrifuged for 15 minutes at 15000 rpm, and the pellet was collected. Then, with ethanol precipitation (70%), the total RNA was obtained.

### (2) Obtaining the expression profiles of about 39000 genes by microarray, and selection of marker genes

In the stool of colon cancer patients, living cells other than bacteria include a small amount of cancer cells, lymphocytes, red blood cells and anal squamous cells. It is presumed that the cells shed from the tunica mucosa coli do not include living cells. In contrast, in the stool of healthy subjects (including those with hemorrhoids), there are lymphocytes, red blood cells and anal squamous cells. Therefore, genes that are expressed in almost all cases of early and advanced colon cancer and that are not expressed in peripheral blood and in squamous cells are potentially good markers for screening of colon cancer from stool. By taking into consideration that there could be very small amounts of living cells from shedding of the tunica mucosa coli, there was an additional condition that the gene not be expressed in the normal tunica mucosa coli, and the number of marker candidates was narrowed. The narrowing was conducted using a genome-wide gene expression analysis using a microarray.

For the microarray, human U133 oligonucleotide probe arrays (Affymetrix, US) were used according to the method recommended by the manufacturer. This will be described briefly. From the 5 µg of total RNA, a cDNA having a T7 RNA polymerase promoter was synthesized. Next, a biotinylated cRNA probe was created by the T7-transcription method. Next, 10 µg of the chemically cleaved cRNA was reacted with the microarray at 45°C for 16 hours. The array was cleaned with 6 × SSPE at 25°C. This was further cleaned with a secondary cleaning solution (100 mM MES (pH 6.7), 0.1 M NaCl, and 0.01% Tween 20) at 50°C. Next, the re-associated molecules were stained with streptavidin phycoerythrin (MolecularProbes) and then washed with 6x SSPE. This was further reacted with biotinylated anti-streptavidin IgG and re-stained with streptavidin phycoerythrin and then cleaned with 6x SSPE. The signal on the microarray was read by GeneArray scanner (made by Affymetrix) at a resolution of 3 µm. The intensities were analyzed using computer software Microarray Suite 5.0 (made by Affymetrix).

Gene expression amounts were analyzed using Microsoft Excel. As a result of selecting genes that were detected in all colon cancer cases but that were not detected in normal tunica mucosa coli and in peripheral blood, 50 types of genes were selected (Table 1). As a result of surveying the 50 genes in a public database (SBM DB: http://www.lsbm.org/db/index.html), these genes were found to have extremely low expression in skin and in squamous cells of the uterine cervix. Therefore, all of these 50 genes had satisfied the requirements that the inventors considered for markers for colon cancer screening. For these 50 genes, specific probes and primers were designed as shown in Table 1 and Table 2.

**[Table 1]**

| No. | Gene name | GenBank ID | probe (5'→3') | Sequence ID No. |
|---|---|---|---|---|
| 1 | PAP | NM_002580 | TTCCCCCAACCTGACCACCTCATTCTTATCTTTCTTCTGTTTCTTCCTCCCCGCTGTCAT | SEQ ID NO: 1 |
| 2 | REG1A | AF172331 | GACCATCTCTCCAACTCAACTCAACCTGGACACTCTCTTCTCTGCTGAGTTTGCCTTGTT | SEQ ID NO: 2 |
| 3 | COL1A1 | Y15916 | GAGGCATGTCTGGTTCGGCGAGAGCATGACCGATGGATTCCAGTTCGAGTATG | SEQ ID NO: 3 |
| 4 | MMP11 | NM_005940 | CATGACAACTGCCGGGAGGGCCACGCAGGTCGTGGTCACCTGCCAGCGACTGTCTC | SEQ ID NO: 4 |
| 5 | SERPINB5 | NM_002639 | CAGGGGCTTCTAGCTGACTCGCACAGGGATTCTCACAATAGCCGATATCAGAATTTGTGT | SEQ ID NO: 5 |
| 6 | DPEP1 | NM_004413 | CAGATGCCAGGAGCCCTGCTGCCCACATGCAAGGACCAGCATCTCCTGAGAG | SEQ ID NO: 6 |
| 7 | DEFA5 | NM_021010 | TATTGCCGAACCGGCCGTTGTGCTACCCGTGAGTCCCTCTCCGGGGTGTGTGAAAT | SEQ ID NO: 7 |
| 8 | TACSTD2 | NM-002353 | ATCTGTATGACAACCCGGGATCGTTTGCAAGTAACTGAATCCATTGCGACATTGTGAAGG | SEQ ID NO: 8 |
| 9 | MMP7 | NM_002423 | ACAGGATCGTATCATATACTCGAGACTTACCGCATATTACAGTGGATCGATTAGTGTCAA | SEQ ID NO: 9 |
| 10 | SLCO4A1 | NM_016354 | CAGCATTCCTGCACTAACGGCAACTCTACGATGTGTCCGTGACCCTCAGAGATC | SEQ ID NO: 10 |
| 11 | SFRP4 | NM_003014 | ACAAACCCGAAAAGAGTGTGAGCTAACTAGTTTCCAAAGCGGAGACTTCCGACTTCCTTA | SEQ ID NO:11 |
| 12 | COL11A1 | J04177 | ACTTGCACGTGTCCCTGAATTCCGCTGACTCTAATTTATGAGGATGCCGAACTCTGATGG | SEQ ID NO: 12 |
| 13 | KRT23 | NM_015515 | GGAACTGACGCAGCTACGCCATGAACTGGAGCGGCAGAACAATGAATACCAAG | SEQ ID NO: 13 |
| 14 | RAB2 | NM_002865 | CCGCGGCCATGGCGTACGCCTATCTCTTCAAGTACATCATAATCGGCGACACA | SEQ ID NO: 14 |
| 15 | KIAA1199 | NM_018689 | TCTGTTGCCGAAATAGCTGGTCCTTTTTCGGGAGTTAGATGTATAGAGTGTTTGTATGTA | SEQ ID NO: 15 |
| 16 | MCM7 | NM_005916 | CAGGACCGGCCCGACCGAGACAATGACCTACGGTTGGCCCAGCACATCACCTAT | SEQ ID NO: 16 |
| 17 | LY6G6D | NM_021246 | GTGCGCTGTGCTAGGTCAGCACCACAACTACCAGAACTGGAGGGTGTACGAC | SEQ ID NO: 17 |
| 18 | G3BP | NM_005754 | GAAGAAGACTCGAGCTGCCAGGGAAGGCGACCGACGAGATAATCGCCTTCGG | SEQ ID NO: 18 |
| 19 | ABHD4 | NM_022060 | CACTGGCCGAGGATAAGCCCGTCCCTGTCCCACATTCTAGCCCCACTATGCG | SEQ ID NO: 19 |
| 20 | POLD2 | NM_006230 | ACTGCAGCGTATCAAACTAAAAGGCACCATTGACGTGTCAAAGCTGGTTACGGGGACTG1 | SEQ ID NO: 20 |
| 21 | TIF1 | NM_003852 | TAATACCACTACCCGTGAATTATATGGCCTGACAATATGAATTAGGTGTACTGTACTGAA | SEQ ID NO: 21 |
| 22 | CYP2B6 | NM_000767 | TAGTCTTCCCCAGTCCTCATTCCCAGCTGCCTCTTCCTACTGCTTCCGTCTATCAAAAAG | SEQ ID NO: 22 |
| 23 | TNK1 | AF097738 | TGGCCACATGGGACCAAGCGGAACCAGAACAAGGTCCCGACAGGGGTAGACGTT | SEQ ID NO: 23 |
| 24 | HSPH1 | NM_006644 | AGTCAAAGTGCGAGTCAACACCCATGGCATTTTCACCATCTCTACGGCATCTATGGTGGA | SEQ ID NO: 24 |
| 25 | NQO1 | NM_000903 | GTCTTAGAACCTCAACTGACATATAGCATTGGGCACACTCCAGCAGACGCCCGAATTCAA | SEQ ID NO: 25 |
| 26 | IRO039700 | BC006214 | TGGGCCCAAGGCTCATGCACACGCTACCTATTGTGGCACGGAGAGTAAGGAC | SEQ ID NO: 26 |
| 27 | FLJ10535 | NM_018129 | CCACCACGCTTGGCCGGGATAGTATATTTTTATAGCACTTCCCCTACTGATTGCTGCCTT | SEQ ID NO: 27 |
| 28 | SLC5A1 | NM_000343 | GAAATATTGCGGTACCAAGGTTGGCTGTACCAACATCGCCTATCCAACCTTAGTGGTGGA | SEQ ID NO: 28 |
| 29 | SYNCRIP | NM_006372 | ATGACGATTACTACTATTATGGTCCACCTCATATGCCCCCTCCAACAAGAGGTCGAGGGC | SEQ ID NO: 29 |
| 30 | AURKB | BC080581 | TATGTCTGTGTATGTATAGGGGAAAGAAGGGATCCCTAACTGTTCCCTTATCTGTTTTCT | SEQ ID NO: 30 |
| 31 | DDX17 | NM_006386 | CTCTGCAAGCTATCGGGATCGTAGTGAAACCGATAGAGCTGGTTATGCTAATGGCAGTGG | SEQ ID NO: 31 |
| 32 | HSPA4 | BC002526 | GCCGGCGGCATCGAGACTATCGCTAATGAGTATAGCGACCGCTGCACGCCGG | SEQ ID NO: 32 |
| 33 | SCN10A | NM_006514 | AAAGGCCTATCGGAGCTATGTGCTGCACCGCTCCATGGCACTCTCTAACACC | SEQ ID NO: 33 |
| 34 | IgH VH | AB035175 | AAGAACACGCTGTATCTGCAAATGCACAGGCTGAGAGCCGAGGACACGGCCGTATA | SEQ ID NO: 34 |
| 35 | MTAP | AF109294 | GCCCGGCGATATTGTCATTATTGATCAGTTCATTGACAGGACTATTTGCCACGACATTTC | SEQ ID NO: 35 |
| 36 | NEK3 | NM_002498 | CCCTCACATCGCCCCTCGGCTACAACGCTTCTCTCTCGAGGCATCGTAGCTCG | SEQ ID NO: 36 |
| 37 | ITGB4 | NM_000213 | GGAGGACTACGACAGCTTCCTTATGTACAGCGATGACGTTCTACGCTCTCCATCGG | SEQ ID NO: 37 |
| 38 | MET | NM_000245 | CTGCCTGACCTTTAAAAGGCCATCGATATTCTTTGCTCCTTGCCATAGGACT1'GTATTGT | SEQ ID NO: 38 |
| 39 | KPNA6 | NM_012316 | CCTTTGTTAACACTCCTTACCAAGTCCACACGACTGACGATGACACGGAATGCAGTCTGG | SEQ ID NO: 39 |
| 40 | PROX1 | NM_002763 | CAGCACCGCCGAAGGGCTCTCCTTGTCGCTCATAAAGTCCGAGTGCGGCGATCTTCAAGf | SEQ ID NO: 40 |
| 41 | WTAP | NM_004906 | GGAAGTTTACGCCTGATAGCCAAACAGGGAAAAAGTTAATGGCGAAGTGTCGAATGCTTA | SEQ ID NO: 41 |
| 42 | FLJ10858 | NM_018248 | AAAGGCCGGATGCTAGGTGATGTGCTAATGGATCAGAACGTATTGCCTGGAGTAGGGAAC | SEQ ID NO: 42 |
| 43 | PHF16 | NM_014735 | AGCCCAGTTGTAGTAGGTGCCAGTCAGTCAAGGCAGGGGCCCTCTCTCCGTCAATA | SEQ ID NO: 43 |
| 44 | TRE5 | X78262 | CATGTTGGCCAAGCTAGTCTCGAACTACTGACTTCGGGTGATCTGCCCTCCTCG | SEQ ID NO: 44 |
| 45 | CI_CGAP_Ut | M27830 | TCGCCCGTCACGCACCGCACGTTCGTGGGGAACCTGGCGCTAAACCATTCGTA | SEQ ID NO: 45 |
| 46 | TRIM31 | AF230386 | CTCAGGATACGAAGACATTTGACGTTGCGCTGTCCGAGGAGCTCCATGCGGCAC | SEQ ID NO: 46 |
| 47 | AP1S1 | NM_001283 | GCTGATCCACCGATACGTGGAGCTCTTAGACAAATACTTTGGCAGTGTGTGCGAGCTGGA | SEQ ID NO: 47 |
| 48 | CYLC2 | NM_001340 | CTCTCAAACCAACTCGTACTGTCGAGGTGGATTCTAAAGCAGCAGAAATTGGTAAGAAAG | SEQ ID NO: 48 |
| 49 | DHX9 | BF313832 | CTCAGAAACGGACGACGCAAGAAGTGCAAGCGACTTCTAGAATTCAGAACCGAAAGTGGf | SEQ ID NO: 49 |
| 50 | REG1B | NM_006507 | AACTGGTCCTGCAATTACTATGAAGTCAAAAATTAAACTAGACTATGTCTCCAACTCAGT | SEQ ID NO: 50 |

**[Table 2]**

| No. | Gene name | Forward-Primer(5'→3') | Sequence ID No. | Reverse-Primer(5'-3') | Sequence ID No. |
|---|---|---|---|---|---|
| 1 | PAP | GAGAAGCACAGCATTTCTGAG | SEQ ID NO: 51 | TGCTCTTTAAAGCCTTAGGCC | SEQ ID NO: 52 |
| 2 | REG1A | AATCCTGGCTACTGTGTGAG | SEQ ID NO: 53 | TCCAAAGACTGGGGTAGGT | SEQ ID NO: 54 |
| 3 | COL1A1 | CTACTACCGGGCTGATGATG | SEQ ID NO: 55 | GGAGGACTTGGTGGTTTTGT | SEQ ID NO: 56 |
| 4 | MMP11 | CACGAATATCAGGCTAGAGAC | SEQ ID NO: 57 | CACATTTACAATGGCTTTGGAG | SEQ ID NO: 58 |
| 5 | SERPINB5 | GGCTCCAGTGAAACTTGG | SEQ ID NO: 59 | CAAGGTAACGTGAGCACTT | SEQ ID NO: 60 |
| 6 | DPEP1 | ACCCATTACGGCTACTCCTC | SEQ ID NO: 61 | AAGGGGTGTTGCTTTTATTGC | SEQ ID NO: 62 |
| 7 | DEFA5 | CAGCCATGAGGACCATC | SEQ ID NO: 63 | TAGAAAGACACAAGGTACACA | SEQ ID NO: 64 |
| 8 | TACSTD2 | GAGAAAGGAACCGAGCTTGT | SEQ ID NO: 65 | TGGTAGTAAGGGCAAGCTGA | SEQ ID NO: 66 |
| 9 | MMP7 | TGGCCTACCTATAACTGGAA | SEQ ID NO: 67 | AATGGATGTTCTGCCTGAAG | SEQ ID NO: 68 |
| 10 | SLCO4A1 | GAAGGCCACCTGAACCTAAC | SEQ ID NO: 69 | CCATCTGAAGACTCCGACAG | SEQ ID NO: 70 |
| 11 | SFRP4 | GATCTTCAAGTCCTCATCACG | SEQ ID NO: 71 | ACCAGCTTTAACTCACCTTC | SEQ ID NO: 72 |
| 12 | COL11A1 | GTACGTCCAGAAAAGGCTATG | SEQ ID NO: 73 | GGACTTAGGGTCATCGGAA | SEQ ID NO: 74 |
| 13 | KRT23 | AGGTGACATCCACGAACT | SEQ ID NO: 75 | GTAGGAAAGTAGAGCTTTACCC | SEQ ID NO: 76 |
| 14 | RAB2 | CAGTTCGTCCGGCTTCCTC | SEQ ID NO: 77 | ATGAAGATGAGTCCATGTTCTCGT | SEQ ID NO: 78 |
| 15 | KIAA1199 | ACTGCACCCATGAGACT | SEQ ID NO: 79 | GTTCATGGTGATGCCTACAA | SEQ ID NO: 80 |
| 16 | MCM7 | GTGGAGAACTGACCTTAGAG | SEQ ID NO: 81 | TCCTTTGACATCTCCATTAGC | SEQ ID NO: 82 |
| 17 | LY6G6D | CTCCTCCTGTTCCTATGTG | SEQ ID NO: 83 | GCAGGAGAAGCATCGATG | SEQ ID NO: 84 |
| 18 | G3BP | AGAGTGCGAGAACAACGAAT | SEQ ID NO: 85 | ACTAAAGGTCAGGAAAGGGAA | SEQ ID NO: 86 |
| 19 | ABHD4 | CTTCACCTGCAGAGTCCTTT | SEQ ID NO: 87 | GATAGAGGGTCATGCAGTGG | SEQ ID NO: 88 |
| 20 | POLD2 | CAACTCCTCACAACCCTTCC | SEQ ID NO: 89 | TTCTTGGTGAGGTATTTGGC | SEQ ID NO: 90 |
| 21 | TIF1 | GAAGAAACGCCTCAAAAGC | SEQ ID NO: 91 | TTTTCTTTAATACAGTTGCCATCT | SEQ ID NO: 92 |
| 22 | CYP2B6 | CAAGCTGTCACTCCCCATAC | SEQ ID NO: 93 | GGGAGGTCAGGCTTTAGAGA | SEQ ID NO: 94 |
| 23 | TNK1 | TGGTTTCTGCCATCCGGAA | SEQ ID NO: 95 | CTTGATCCTCTCTAGCGCGTAA | SEQ ID NO: 96 |
| 24 | HSPH1 | CAATACTTTCCCCGGCAT | SEQ ID NO: 97 | CCTAACTGCCAGACCAAG | SEO ID NO: 98 |
| 25 | NQO1 | CGCAGACCTTGTGATATTCC | SEQ ID NO: 99 | CGATTCCCTCTCATTTATTCCTT | SEQ ID NO: 100 |
| 26 | IRO639700 | GAGGATGATGAGCTGCTACA | SEQ ID NO: 101 | CTACACACTTTTATTGGAGGGG | SEQ ID NO: 102 |
| 27 | FLJ10535 | AACTCATTTACGGATAGGACTTT | SEQ ID NO: 103 | TTCCTGTCCTATTGGTACCA | SEQ ID NO: 104 |
| 28 | SLC5A1 | AAATGCTACACTCCAAGGGC | SEQ ID NO: 105 | CAGAAAATAGCAAGCAGGAAG | SEQ ID NO: 106 |
| 29 | SYNCRIP | AATGGGCTGATCCTATAGAAGA | SEQ ID NO: 107 | CTCTTTGTTGTTGGGCACCT | SEQ ID NO: 108 |
| 30 | AURKB | ACCTCATCTCCAAACTGCTCA | SEQ ID NO: 109 | AAAAAGCTTCAGCCTTTATTAAACA | SEQ ID NO: 110 |
| 31 | DDX17 | CTCAGAGGATTATGTGCACCG | SEQ ID NO: 111 | AGGAGGAGGAGGGTATTGGT | SEQ ID NO: 112 |
| 32 | HSPA4 | CAGTACCCACTGGAAGGACTTA | SEQ ID NO: 113 | TCTCCTTCAGTTTGGACAAAAG | SEQ ID NO: 114 |
| 33 | SCN10A | AGAACTTCAATGTGGCCACG | SEQ ID NO: 115 | CATACTGGTGGCTTCATCTT | SEQ ID NO: 116 |
| 34 | 1gH VH | GTGGGTCTCAGCTATTAGTGG | SEQ ID NO: 117 | GGATTTCGCACAGTAATATACGG | SEQ ID NO: 118 |
| 35 | MTAP | CTCGCTTGGTTCCCTTAGTC | SEQ ID NO: 119 | ATCCCTGCAGGAAAATCAT | SEQ ID NO: 120 |
| 36 | NEK3 | ATGTCAAGGGTGCATCAGTC | SEQ ID NO: 121 | GAACCCTTCTGAACCTGGTC | SEQ ID NO: 122 |
| 37 | ITGB4 | TCTGGCCTTCAATGTCGTCT | SEQ ID NO: 123 | TGTGGTCGAGTGTGAGTGTT | SEQ ID NO: 124 |
| 38 | MET | ATGTCCATGTGAACGCTACT | SEQ ID NO: 125 | CCAAGCCTCTGGTTCTGATG | SEQ ID NO: 126 |
| 39 | KPNA6 | CAAGAGTGGTGGATCGGTTC | SEQ ID NO: 127 | TCAACAAGTGGAGAAGGCAA | SEQ ID NO: 128 |
| 40 | PROX1 | TGATGGCCTATCCATTTCAG | SEQ ID NO: 129 | AACATCTTTGCCTGCGATAA | SEQ ID NO: 130 |
| 41 | WTAP | AAGCAACAACAGCAGGAGTC | SEQ ID NO: 131 | TGTGAAATCCAGACCCAGAC | SEQ ID NO: 132 |
| 42 | FLJ10858 | ATTTCGGAATGAAAGGCTTC | SEQ ID NO: 133 | CCCTGCTAGATGTCCAACTG | SEQ ID NO: 134 |
| 43 | PHF16 | GCTTATACCCTGTTCCCAAA | SEQ ID NO: 135 | ACACAGCTCCATCATAATTTCAT | SEQ ID NO: 136 |
| 44 | TRE5 | GCTCAGTGCTAGTCTGTTGTGTAG | SEQ ID NO: 137 | CCCTGGCCTCAAGTGATCC | SEQ ID NO: 138 |
| 45 | CI_CGAP_U | GAATTCACCAAGCGTTGGA | SEQ ID NO: 139 | GCTGACTTTCAATAGATCGCAG | SEQ ID NO: 140 |
| 46 | TRIM31 | TGTTCCTCTGGAACTGGAGA | SEQ ID NO: 141 | CCCTCCTTTTGCTCAAGAAT | SEQ ID NO: 142 |
| 47 | AP1S1 | GAAAGCCCAAGATGTGCAG | SEQ ID NO:143 | GAGTCCCACCAAGAACAGG | SEQ ID NO: 144 |
| 48 | CYLC2 | AGAGTAAACTTTGGGCCATATGA | SEQ ID NO: 145 | ACCTTTTTTGCTATCTTTCTCTGT | SEQ ID NO: 146 |
| 49 | DHX9 | CTTGAATCATGGGTGACGTT | SEG ID NO: 147 | GTTTGGTGCGATTATGTGGT | SEQ ID NO: 148 |
| 50 | REG1B | CTCAGGATTCAAGAAATGGAAGG | SEQ ID NO: 149 | GTGAAGGTACTGAAGATCAGCG | SEQ ID NO: 150 |

### Example 2

### Optimization of the PCR conditions for the 50 selected genes

### (1) Reverse transcription (first strand synthesis)

With 5 µg of the total RNA of the advanced colon cancer tissue obtained in Example 1, reverse transcription was conducted with a random hexamer primer using Superscript Choice System from Invitrogen. The following is a more concrete description of the method.

The total RNA was adjusted to a concentration of 10 µg/10 µl. To this, 1 µl of random hexamer primer was added. This was heat denatured by incubation at 68°C for 10 minutes. This was rapidly cooled by placing on ice for 2 minutes or greater. Next, the reagents shown in Table 3 were added. This was incubated for 25°C for 10 minutes, 42°C for 60 minutes and 68°C for 15 minutes. Afterwards, this was cooled rapidly and after spinning down, 1 µl of RNase H was added, and this was maintained at 37°C for 20 minutes. In this way, approximately 20 µl of 1st strand cDNA solution was recovered.

**[Table 3]**

| Reagent | Amount to be added |
|---|---|
| 5×1st strand buffer | 4 µl |
| 10 mM dNTP | 1 µl |
| 0.1 M DTT | 2 µl |
| RNase inhibiter | 0.5 µl |
| SuperScript II RT | 1 µl |

### (2) PCR amplification

Using the recovered 1st strand cDNA solution as a template, PCR amplification of the 50 genes described in Table 1 was conducted. For the template, in each PCR reaction, a 3 times dilution of the 1st strand cDNA solution of the colon cancer tissue synthesized in (1) was used. For the primer, the primer set described in Table 2 was used. As the PCR reaction solution, the PCR enzyme Takara Ex Taq from Takara Bio was used, and the solution was prepared with the composition shown in Table 4.

**[Table 4]**

| Reaction mixture composition | |
|---|---|
| Reagent | Amount to be added |
| Takara Ex Taq | 0.5 µl (2.5U) |
| 10× Ex Buffer (20 mM Mg²⁺) | 5.0 µl |
| Template cDNA | 1.0 µl |
| Forward Primer (F) (10 µM) | 2.5 µl (25 pmol/tube) |
| Reverse Primer (R) (10 µM) | 2.5 µl (25 pmol/tube) |
| dNTP Mix | 2.5 µl (200 µlM) |
| Distilled water | 36.0 µl |
| Total | 50 µl |

For the reaction solution that was prepared, PCR amplification reaction was conducted using a commercially available thermocycler according to the temperature cycle protocol of Table 5. After completion, the reaction solution was stored at 4°C.

**[Table 5]**

| Temperature condition for PCR amplification | | | | |
|---|---|---|---|---|
| Step | Temperature | | Holding time | Repeat No. |
| 1 | 95°C | | 5 min. | |
| 2 | 95°C | (denaturation) | 30 sec. | 30 cycles |
| 3 | 58°C | (annealing) | 30 sec. | |
| 4 | 72°C | (extension) | 40 sec. | |
| 5 | 72°C | | 10 min. | |

### (3) Electrophoresis

Using 10 µl from each of the resulting PCR products, 1.5% agarose gel electrophoresis was conducted, and this was stained with EtBr solution. As a result, for each PCR product, a single thick band at a desired chain length was detected, hence it was clearly shown that there was one main product.

As seen in the experiments of (1) through (3) described above, by extracting the total RNAs from colon cancer cells according to the usual methods, and by conducting RT-PCR using the designed primers, amplification of the targeted amplification region was certainly performed.

### Example 3

### (Selection step 2) Secondary selection by RT-PCR of the 50 genes

Cells separated from stool by MACS (magnetic cell sorting) which uses epithelial cell specific antibody (Dynabeads Epithelial Enrich, Invitrogen International) (this is described in detail in 1 of Example 6) hardly contain any lymphocytes or red blood cells. As a result, the 50 genes selected in the selection step 1 are genes that are effective for colon cancer screening of these separated cells. On the other hand, in order to conduct screening by extracting RNA directly from stool, a further selection for genes which are not detected in lymphocytes and red blood cells is needed.

As with Example 1, total RNA was extracted from another 22 cases of Dukes' A, B and 8 cases of Dukes' C, D and from peripheral blood. In order to understand the characteristics of the 50 genes, RT-PCR was conducted by the following method.

### (1) Reverse transcription reaction (synthesis of single stranded cDNA)

Using Superscript Choice System from Invitrogen, reverse transcription of 5 µg of the total RNA with a random hexamer primer was conducted. Stated more concretely, the following method was used.

The total RNA was adjusted to a concentration of 10 µg/10 µl. To this, 1 µl of random-hexamer primer was added. This was heat denatured by incubating at 68°C for 10 minutes. After rapid cooling by placing on ice for 2 minutes or greater, the reagents shown above in Table 3 were added. This was incubated at 25°C for 10 minutes, 42°C for 60 minutes, and 68°C for 15 minutes. Afterwards, this was rapidly cooled and spun down. Next, 1 µl of RNase H was added, and this was maintained at 37°C for 20 minutes. With this method, approximately.20 µl of 1st strand cDNA solution was recovered.

### (2) PCR amplification

Using the recovered 1st strand cDNA solution as a template, PCR amplification of the 50 genes listed in Table 1 was conducted. For the template, in each PCR reaction, a three times dilution of the 1st strand cDNA solution of the colon cancer cell from (1) was used. For the primer, the primer set described in Table 2 was used.

For the PCR reaction, PCR kit Takara Ex Taq from Takara Bio was used, and the reaction solution shown above in Table 4 was prepared. For the prepared reaction solution, a commercially available thermocycler was used. The PCR amplification reaction was conducted according to the temperature cycle protocol of the above Table 5. After completing the PCR, the reaction solution was stored at 4°C.

### (3) Electrophoresis

Using 10 µl of each of the resulting PCR product, 1.5% agarose gel electrophoresis was conducted, and this was stained with EtBr solution.

### (4) Experiment results

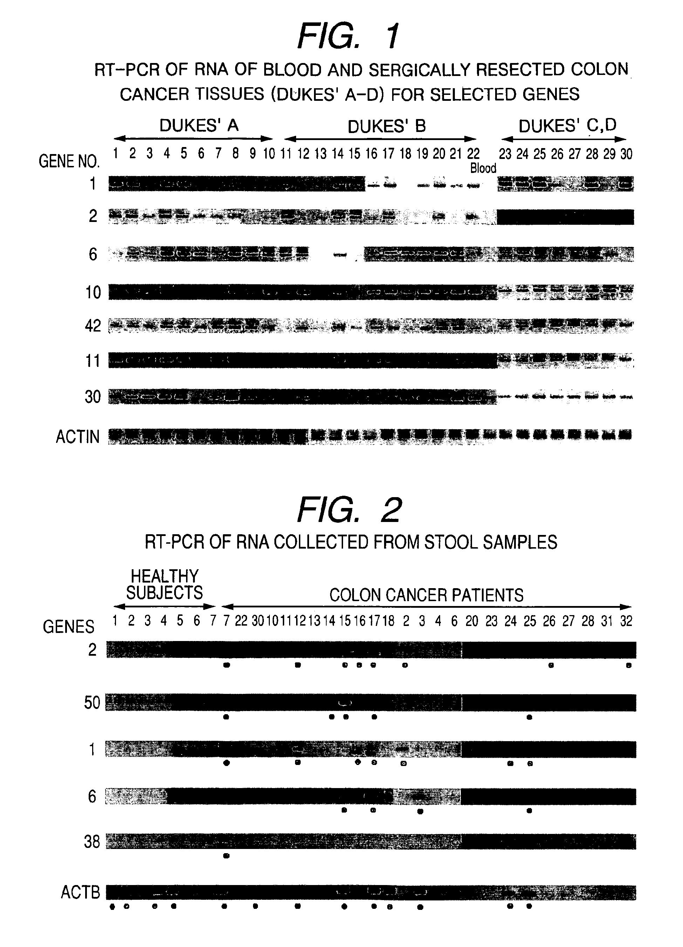

Of the 50 genes, there were 15 genes which were not detected in peripheral blood (genes No. 1, 2, 6, 8, 10, 11, 25, 30, 37, 38, 40, 41, 42, 46, 50). Of these, electrophoresis results of representative genes (No. 1, 2, 6, 10, 11, 30, and 42) are shown in FIG. 1. In addition, in Table 6, the,presence or absence of expression of all 15 genes in the 30 cases of colon cancer tissue is shown. In both early cancer (Dukes' A, B) and advanced cancer (Dukes' C, D), expression was observed in 70-100% of cases. In all cases, expression of a plurality (7-15) of genes was observed.

These 15 genes were not detected in peripheral blood after 30 cycles of PCR, and even with a further 20 cycles. From these results, we concluded that these were marker genes which can differentiate colon cancer from hemorrhoids. The 15 genes described above and their probes and primers used in the present invention are summarized as shown in Tables 26 and 27.

**[Table 6]**

| Expression of the 15 genes not detected in the blood in 30 cases of colon cancer tissues | | | | | | | | | | | | | | | |
|---|---|---|---|---|---|---|---|---|---|---|---|---|---|---|---|
| 15 genes | | | | | | | | | | | | | | | |
| | 1 | 2 | 6 | 10 | 42 | 11 | 30 | 8 | 25 | 37 | 38 | 40 | 41 | 46 | 50 |
| Samples | | | | | | | | | | | | | | | |
| Dukes' A (n=10) | 90 | 90 | 100 | 90 | 90 | 100 | 100 | 100 | 100 | 90 | 100 | 100 | 100 | 90 | 100 % |
| Dukes' B (n=12) | 83 | 83 | 92 | 100 | 100 | 83 | 100 | 100 | 100 | 92 | 100 | 100 | 92 | 42 | 100 % |
| Dukes' C, D (n=8) | 75 | 88 | 100 | 100 | 100 | 100 | 100 | 100 | 100 | 100 | 100 | 100 | 100 | 88 | 100 % |
| Total (n=30) | 83 | 87 | 97 | 97 | 97 | 93 | 100 | 100 | 100 | 93 | 100 | 100 | 97 | 70 | 100 % |
| Blood (58 mix) | (-) | (-) | (-) | (-) | (-) | (-) | (-) | (-) | (-) | (-) | (-) | (-) | (-) | (-) | (-) |

### Example 4

### Expression analysis by DNA microarray

### I. Preparation of DNA microarray

### (1) Cleaning of glass substrate

A synthetic quartz glass substrate (size (WxLxT): 25 mm × 75 mm × 1 mm, Iiyama Precision Glass) was placed in a heat-resistant and alkali-resistant rack. A cleaning solution for ultrasonic cleaning was prepared at a prescribed concentration, and the glass substrate was immersed in this cleaning solution. After immersing in the cleaning solution overnight, ultrasonic cleaning was conducted for 20 minutes. Next, the glass substrate was taken out, then rinsed lightly with pure water, and subjected to ultrasonic cleaning with ultrapure water for 20 minutes. Thereafter, the glass substrate was immersed for 10 minutes in 1 N sodium hydroxide solution which was heated to 80°C and again cleaned with pure water and ultrapure water. Thus, a cleaned quartz glass substrate for use in DNA chip was prepared.

### (2) Surface treatment

A silane coupling agent KBM-603 (made by Shin-Etsu Chemical) was dissolved in pure water to achieve a concentration of 1%. This was stirred for 2 hours at room temperature. Subsequently, the cleaned quartz glass substrate was immersed in the silane coupling agent solution, and this was left for 20 minutes at room temperature. The glass substrate was pulled out, and after cleaning the surface lightly with pure water, both surfaces of the glass substrate were dried by blowing nitrogen gas. Next, the glass substrate dried by nitrogen blowing was baked for 1 hour in an oven heated to 120°C, and the coupling agent treatment was completed. By this coupling agent treatment, amino groups derived from the silane coupling agent was introduced onto the glass substrate surface.

An EMCS solution was prepared by dissolving N-(6-Maleimidocaproyloxy)succinimide (abbreviated as EMCS) made by DOJINDO in a 1:1 mixture solvent of dimethylsulfoxide and ethanol to achieve a final concentration of 0.3 mg/ml. After completion of the baking, the coupling agent treated glass substrate was cooled and then was immersed for two hours in the EMCS solution at room temperature. During this immersion treatment, the amino group, which was introduced onto the glass substrate surface by the coupling agent treatment, reacted with the succinimide group of EMCS, and the maleimide group from EMCS was introduced onto the surface of the glass substrate. The glass substrate was pulled out of the EMCS solution and was washed using the mixture solvent of dimethylsulfoxide and ethanol described previously. This was further cleaned with ethanol and then dried under a nitrogen gas atmosphere.

### (3) Synthesis of probe DNA

Each of the probe DNA (SEQ ID NOs: 1-50) for detecting the 50 genes shown in Table 1 was synthesized.

In order to have a covalent bond between the probe DNA and the maleimido group which was introduced onto the glass substrate as described above, thiol treatment of the 5' terminus of the probe DNA was performed according to the standard method. Afterwards, in order to avoid side reactions during DNA synthesis, the protective group was deprotected, and further HPLC purification and desalting treatment were performed. Each of the resulting probe DNA was dissolved in pure water and aliquoted so that the final concentration (when dissolved in the ink) would be 10 µM. Freeze-drying was then conducted to remove the water content.

### (4) Probe DNA ejection by BJ printer and bonding to the substrate surface

An aqueous solution containing 7.5 wt% glycerin, 7.5 wt% thiodiglycol, 7.5 wt% urea, 1.0 wt% acetylenol EH (made by Kawaken Fine Chemicals) was prepared. Next, the aliquoted probe DNA was dissolved in this mixture solvent to a prescribed concentration (10 µM). An ink tank for a bubble jet printer (product name: BJF-850 by Canon), was filled with the resulting probe DNA solution, and this was attached to the printer head.

The bubble jet printer was modified to accommodate ink jet printing onto a flat surface. In addition, with this modified bubble jet printer, by inputting a printing pattern according to a prescribed file creation method, DNA solution droplets of approximately 5 pl can be spotted at a pitch of approximately 190 µm.

Next, using the modified bubble jet printer, spotting operation of the probe DNA solution onto the glass substrate surface was performed. A printing pattern was created beforehand so that 16 spots would be ejected for each probe onto one DNA microarray. Thus, ink jet printing was conducted. Using a magnifying glass or the like, spotting of the DNA solution in the desired pattern was confirmed. Next, this was placed in a humidified chamber for 30 minutes at normal temperature. The maleimido group of the glass substrate surface and the sulfanyl (-SH) group on the 5' terminus of the probe DNA were reacted.

### (5) Cleaning

After reacting for 30 minutes in the humidified chamber, any unreacted probe DNA remaining on the glass substrate surface was washed away with 100 mM NaCl containing 10 mM phosphate buffer solution (pH 7.0). A DNA microarray was obtained in which the prescribed single stranded probe DNA was fixed onto the glass substrate surface at 16 spots per DNA chip.

### II. Hybridization reaction

### (1) Amplification of sample and labeling (PCR Amplification with incorporation of label)

For the sample, the 1st strand cDNA solution synthesized in Example 1 was used. Of 50 genes in Table 1, for 10 of these genes which are shown in Table 9, PCR amplification with label incorporation was conducted using the 1st strand cDNA as the template. For the primer, the primer set shown in Table 2 was used. The PCR reaction was conducted by preparing the reaction solution shown in Table 7 using the PCR enzyme Takara Ex Taq made by Takara Bio. The solution was prepared so that the final concentration of dNTP was 200 µM.

**[Table 7]**

| Reaction mixture composition | |
|---|---|
| Reagent | Amount to be added |
| Takara Ex Taq | 0.5 µl (1.0U) |
| 10× Ex Taq Buffer (20 mM Mg²⁺) | 5.0 µl |
| Template DNA (cDNA solution) | 1. 0 µl |
| Forward Primer (F) (10 µM) | 2.5 µl (25 pmol/tube) |
| Reverse Primer (R) (10 µM) | 2.5 µl (25 pmol/tube) |
| dNTP Mixture (*) | 2.0 µl |
| Cy3 dUTP (1.0 mM, Amersham Biosciences) | 2.0 µl (40 µM) |
| Distilled water | 34.5 µl |
| Total | 50 µl |

| | |
|---|---|
| (*) Concentration: 5.0 mM for dATP, dCTP and dGTP; 4.0 mM for dTTP | |

With regard to the prepared reaction solution, a commercially available thermocycler was used to conduct PCR amplification reaction according to the temperature cycle protocol seen in Table 5. After completion, the reaction solution was stored at 4°C.

After the reaction was completed, the reaction solution was purified with a purification column (Qiagen Co. QIAquick PCR Purification Kit). After eluting with 50 µl of distilled water, the resulting purified product was the labeled sample.

Using the DNA microarray created in I and the 10 labeled samples, hybridization was conducted on the microarray.

### (2) Blocking of the DNA microarray

BSA (bovine serum albumin Fraction V: made by Sigma) was dissolved in 100 mM NaCl / 10 mM Phosphate Buffer to a concentration of 1 wt%. The DNA microarray prepared in II was immersed in this solution for 2 hours at room temperature, and blocking of the glass substrate surface was conducted. After blocking was completed, the DNA microarray was cleaned with a 0.1x SSC solution (15 mM of NaCl, 1.5 mM of sodium citrate (trisodium citrate dihydrate C₆H₅Na₃-2H₂O), pH 7.0) containing 0.1 wt% SDS (sodium dodecyl sulfate). Next, this was rinsed with pure water. Next, the DNA microarray was dewatered with a spin dry apparatus.

### (3) Preparation of hybridization solution

The hybridization solution was prepared for each PCR product so that the final concentration was 6x SSPE / 10% Formamide / PCR amplification product solution (6x SSPE: 900 mM of NaCl, 60 mM of NaH₂PO₄-H₂O, 6 mM of EDTA, pH 7.4). For each PCR amplification product solution, 25.0 µl, which is approximately half of the purified product, was used.

### (4) Hybridization

The dewatered DNA chip was set on a hybridization apparatus (Hybridization Station from Genomic Solutions Inc.). Using the hybridization solution with the above described composition, the hybridization reaction was conducted with the procedure and conditions shown in Table 8.

**[Table 8]**

| Conditions and procedures for hybridization | |
|---|---|
| Operation | Operation procedures and conditions |
| Reaction | 65°C 3 min → 92°C 2 min → 55°C 4 h |
| Washing | 2× SSC / 0.1% SDS at 25°C |
| | 2× SSC at 20°C |
| (Rinse) | Distilled water (manual rinse washing) |
| Drying | Spin dry |

### (5) Fluorescence measurement

After completion of the hybridization reaction, the fluorescence of the hybrid on the DNA chip that had been spun dry was measured using DNA microarray fluorescence detection apparatus (Genepix 4000B made by Axon). The results for the measured fluoroluminance are shown in Table 9.

For the calculation of luminance, first, the actual measured value of the fluorescent intensity was calculated by subtracting a background value from the apparent fluorescent intensity from each spot. Then, the fluoroluminance value was calculated as an average value for the 16 spots. The background value was the fluorescent intensity that was seen on the DNA chip in areas where there was no probe DNA spot.

As is clear from these results, the expression of each gene has an adequate signal and can be measured. Similar experiments were conducted with other genes. With all of the probes and primers, gene expression analysis that is specific and highly sensitive was possible.

**[Table 9]**

| No. | Gene name | Fluoroluminescence |
|---|---|---|
| 1 | PAP | 1286.9 |
| 2 | REGIA | 1089.3 |
| 6 | DPEP1 | 286.2 |
| 10 | SLCO4A1 | 5814.5 |
| 30 | STK12 | 271.9 |
| 37 | ITGB4 | 3729.6 |
| 38 | MET | 8321.3 |
| 41 | WTAP | 1181.2 |
| 42 | FLJ10858 | 1921.1 |
| 46 | TR1M31 | 1162.9 |

### Example 5

### Expression analysis with the DNA microarray of the gene which has been amplified by multiplex RT-PCR

### (1) Amplification and labeling of the sample.

### (Multiplex PCR amplification with label incorporation)

For the samples, colon cancer tissue from advanced colon cancer, normal tunica mucosa coli, and blood were collected. With regard to each sample, the total RNA was recovered and synthesis of the 1st strand was conducted by the procedure indicated in Examples 1 and 2. In order to eliminate individual differences, the samples collected from 7 patients were mixed. With the resulting 1st strand cDNA solution as a sample, gene amplification and labeling reaction are shown below. In the present example, the 10 genes which have been selected in Example 4 were the targets. With regard to the primers, all 10 types of primers are added to one PCR tube. In other words, multiplex PCR was conducted. For the substrate, Cy3-dUTP was added as in Example 4, and labeling of the PCR product was conducted. The solution composition of the PCR reaction is as shown in Table 10.

**[Table 10]**

| Reaction mixture composition | |
|---|---|
| Reagent | Amount to be added |
| Takara Ex Taq | 2.5 µl (2.5U) |
| 10× Ex Taq Buffer (20 mM Mg²⁺) | 5.0 µl |
| Template DNA (cDNA solution) | 1.0 µl |
| Forward Primer (F) | 12.5 pmol/each |
| Reverse Primer (R) | 12.5 pmol/each |
| dNTP Mixture (*) | 2.0 µl |
| Cy3 dUTP (1.0 mM, Amersham Biosciences) | 2.0 µl (40 µM) |
| Distilled water | Optional |
| Total | 50 µl |

| | |
|---|---|
| (*) Concentrations are the same as shown in Table 7. | |

For the reaction solution that was prepared, a commercially available thermocycler was used to conduct PCR amplification reaction according to the temperature cycle protocol of Table 5 as described in Example 2. After completion, the reaction solution was stored at 4°C.

After the reaction was completed, the reaction solution was purified with a purification column (Qiagen Co. QIAquick PCR Purification Kit). After eluting with 50 µl of distilled water, the resulting purified product was the labeled sample. Using the DNA microarray created in I of Example 4 and these three types of labeled samples, hybridization was conducted on the microarray by the method indicated in Example 4. The fluoroluminance values for each probe are shown in Table 11.

**[Table 11]**

| No. | Gene name | Fluoroluminescence | | |
|---|---|---|---|---|
| | | Colon cancer | Normal tissue | Blood |
| 1 | PAP | 516.5 | 47.1 | 0.0 |
| 2 | REG1A | 110.3 | 23.8 | 0.7 |
| 6 | DPEP1 | 152.9 | 132.2 | 6.5 |
| 10 | SLCO4A1 | 1025.1 | 236.2 | 0.0 |
| 30 | AURKB | 257.6 | 62.2 | 0.0 |
| 37 | ITGB4 | 2561.2 | 738.2 | 0.0 |
| 38 | MET | 5019.1 | 599.8 | 0.0 |
| 41 | WTAP | 1134.7 | 630.1 | 0.0 |
| 42 | FLJ10858 | 316.3 | 86.4 | 0.0 |
| 46 | TRIM31 | 88.0 | 0.0 | 0.0 |

As is clear from these results, even if the sample preparation is conducted by multiplex PCR, the expression for each gene has an adequate signal and can be measured.

In addition, each of the genes is expressed strongly in colon cancer and has a low expression amount in normal tissue. In blood, the genes were either hardly expressed at all, or there was a difference in the fluoroluminance value as compared to colon cancer cells. It was confirmed that even if blood is mixed in the sample, colon cancer diagnosis is still possible.

### Example 6

### RT-PCR analysis using the cells isolated from stool of colon cancer patients

RT-PCR analyses were carried out using RNA from cells isolated from stools of 7 normal subjects and 25 colon cancer patients for the 5 genes (No. 1, 2, 6, 38, and 50 that are PAP, REG1A, DPEP1, MET and REG1B, respectively). The 5 genes described above, and their probes and primers are shown altogether in Table 28 and 29.

### (1) Isolation of cells from stool

Stool from colon cancer patients before operation was used as a sample. For using stool, we explained to patients details of the experiment beforehand and obtained the consent.

Two hundred ml of Hanks solution (Nissui, Nissui Pharmaceutical) containing 10% FBS was added to a stomacher bag containing stool (about 5-80g), and after sealing, the stool suspension was prepared using a stomacher (200 rpm, 1 min).

When a stomacher bag with a filter was used, the suspension was filtered through the filter in the bag. When a stomacher bag without a filter was used, the suspension was filtered through a funnel type filter set on a cylinder shaped plastic container, and the filtrate was collected in a beaker. The filtrate was aliquoted to five 50 ml centrifuge tubes.

Forty µl of Ber-EP4 antibody bound magnetic beads (Dynabeads Epithelial Enrich, Invitrogen International) was added to each of the centrifuge tubes and stirred using a mix rotor (VMR-5, ASONE Co., Ltd.) (4°C, 60 rpm, 30 minutes) to bind cells in the filtrate to Ber-EP4 antibody.

Each of the centrifuge tubes was set to a magnetic stand (Dynal MPC-1, Invitrogen International), placed sideways on a mild mixer (SI-36, TAITEC Co., Ltd.) and moved in a seesaw-like motion for 15 minutes (60 rounds/minute) to stir the filtrate and to collect magnetic beads to the side wall of the centrifuge tube.

After removing the filtrate, the centrifuge tubes were taken out of the stand, and 500 µl of Hanks solution containing 10% FBS was added to each tube to wash the beads collected on the wall of the tube.

The wash solution containing the beads was recovered into 5 microtubes (1.5 ml, made by Eppendorf), each of which contained 500 µl of Hanks solution containing 10% FBS. The beads were suspended lightly, and then the microtubes were set on a magnetic stand (Dynal MPC-S, Invitrogen International) to collect the beads to the side wall of the microtube.

After removing the wash solution, microtubes were taken out of the stand, and 1 ml of Hanks solution containing 10% FBS was added to each tube and the beads collected on the wall of the microtubes were washed. Similarly, the tubes were set on the magnetic stand, the magnetic beads were collected on the side wall of the microtubes and pellets of cell-beads complex were obtained after removing the supernatant. Subsequently RNA was extracted from these pellets using ISOGEN (Nippon Genes).

### (2) RT-PCR analysis

### (i) cDNA synthesis (the first round)

One µg of total RNA obtained as above was subjected to reverse transcription using oligo (dT) primer and SUPERSCRIPT Choice System (Invitrogen). Total RNA (10 µl) was mixed with 1 µl of 100 µM T7-oligo dT 24 primer (1 µg) and incubated at 65°C for 10 minutes. Subsequently, the mixture was rapidly cooled by placing on ice for 2 minutes or longer, and then mixed with reagents shown in Table 12 and incubated at 37°C for 2 minutes.

**[Table 12]**

| Reagent | Amount to be added |
|---|---|
| 5× 1st strand buffer | 4 µl |
| 10 mM dNTP | 1 µl |
| 0.1 M DTT | 2 µl |
| RNase inhibiter | 0.5 µl |

Then the reaction mixture was mixed with 1 µl of SuperScriptII RT and incubated at 37°C for 1 hour. Thus, about 20 µl of the 1^{st} strand cDNA solution was recovered.

Next, the 2^{nd} strand cDNA was synthesized by the method described below. The 1^{st} strand cDNA solution was mixed with reagents as shown in Table 13 and incubated at 16°C for 2 hours.

**[Table 13]**

| Reagent | Amount to be added |
|---|---|
| Distilled water | 91 µl |
| 5×2nd strand buffer | 30 µl |
| 10 mM dNTP | 3 µl |
| E. coli DNA Ligase | 1 µl |
| E. coli DNA polymerase | 4 µl |
| E. coli RNase H | 1 µl |

Further, 2 µl of T4 DNA polymerase was added and incubated at 16°C for 5 minutes to make the ends of the 2^{nd} strand cDNA smooth. Next, the 2^{nd} strand cDNA was purified. The product described above was mixed with reagents shown in Table 14 and centrifuged at 15,000 rpm for 10 minutes.

**[Table 14]**

| Reagent | Amount to be added |
|---|---|
| Glycogen (20 mg/ml) | 1 µl |
| Phenol | 150 µl |

Subsequently, 150 µl of chloroform was added, and the mixture was collected and centrifuged at 15,000 rpm for 10 min, and only the supernatant was collected anc transferred to another tube. Further, reagents were added as shown in Table 15, and the mixture was stand at room temperature for 15 minutes, centrifuged at 15,000 rpm for 10 minutes, and only the supernatant was collected and transferred to another tube.

**[Table 15]**

| Reagent | Amount to be added |
|---|---|
| 7.5 M ammonium acetate aqueous solution | 75 µl |
| Isopropanol | 500 µl |

And then 500 µl of 70% ethanol was added and the mixture was centrifuged at 15,000 rpm for 10 minutes (ethanol rinse), and at this time the precipitates were kept and the solution was discarded. To the remaining precipitates, reagents were added as shown in Table 16 and the mixture was allowed to stand at room temperature for 15 minutes, centrifuged at 15,000 rpm for 10 minutes, and the solution was discarded while the precipitates were kept. To the remaining precipitates, 500 µl of 70% ethanol was added, the mixture was centrifuged at 15,000 rpm for 10 minutes and the solution was discarded. Finally the precipitates were air dried and dissolved in 8 µl of water.

**[Table 16]**

| Reagent | Amount.to be added |
|---|---|
| Distilled water | 100 µl |
| 7.5 M ammonium acetate aqueous solution | 50 µl |
| Isopropanol | 300 µl |

### (ii) Synthesis of cRNA: in vitro transcription (the first round)

Following reaction was carried out using MEGAscript T7 Kit (Ambion). To the 8 µl of cDNA solution prepared in (i), reagents were added as shown in Table 17 and the mixture was incubated at 37°C for 5 hours. Next, 1 µl of DNase (RNase free) was added and the mixture was incubated at 37°C for 15 minutes to remove DNA.

**[Table 17]**

| Reagent | Amount to be added |
|---|---|
| NTP mix | 8 µl |
| 10× T7 RNA polymerase Buffer | 2 µl |
| T7 RNA polymerase enzyme mix | 2 µl |

Subsequently, cRNA was purified. Reagents were added as shown in Table 18 and the mixture was centrifuged at 15,000 rpm for 5 minutes, further mixed with 300 µl of isopropanol, incubated at room temperature for 15 minutes, centrifuged at 15,000 rpm for 10 minutes, and only the supernatant was collected and transferred to another tube. Then the supernatant was mixed with 500 µl of 70 % ethanol, centrifuged at 15,000 rpm for 5 minutes, and only the precipitates were kept, air dried and dissolved in 8 µl of water, while the solution was discarded.

**[Table 18]**

| Reagent | Amount to be added |
|---|---|
| Isogene (Nippon Gene) | 400 µl (5 fold dilution) |
| Chloroform | 100 µl |

### (iii) cDNA synthesis (2^{nd} round)

The second reverse transcription reaction was carried out for cRNA solution obtained as above using random hexamer primers. One µl of 0.5 µg/µl random hexamer (1 µg) was added, and the mixture was incubated at 65°C for 10 minutes. Then, after rapidly cooling by placing on ice for 2 minutes or longer, reagents were added as shown in Table 12 and the mixture was incubated at 37°C for 2 minutes. Then, 1 µl of Superscript II RT was added and the mixture was incubated at 37°C for 1 hour. Thus, about 20 µl of the 1^{st} strand cDNA solution was recovered. Subsequently, RNA was removed by adding 1 µl of RNase H. The mixture was incubated at 37°C for 20 minutes, then at 95°C for 2 minutes to separate RNA and DNA and thereafter rapidly cooled by placing on ice for 2 minutes or longer.

Next, the 2^{nd} strand cDNA was synthesized by the method shown below. The 1^{st} strand cDNA solution was mixed with 1 µl of 100 µM T7-oligo dT 24 primer (1 µg) and incubated at 68°C for 5 minutes and then at 42°C for 10 minutes. Reagents were added as shown in Table 19, and the mixture was incubated at 16°C for 2 hours. Further, 2 µl of T4 DNA polymerase was added and the mixture was incubated at 16°C for 5 minute to make the ends of the 2^{nd} strand cDNA smooth. Next, the 2^{nd} strand cDNA was purified.

**[Table 19]**

| Reagent | Amount to be added |
|---|---|
| Distilled water | 91 µl |
| 5 × 2nd strand buffer | 30 µl |
| 10 mM dNTP | 3 µl |
| E. coli DNA polymerase | 4 µl |
| E. coli RNase H | 1 µl |

To the product described above, 150 µl of phenol was added and the mixture was centrifuged at 15,000 rpm for 10 minutes. Subsequently, 150 µl of chloroform was added, and the mixture was centrifuged at 15,000 rpm for 10 minutes, and only the supernatant was collected and transferred to another tube. Further reagents were added as shown in Table 15 and the mixture was incubated at room temperature for 15 minutes, centrifuged at 15,000 rpm for 10 minutes, and only the supernatant was collected and transferred to another tube. And then 500 µl of 70% ethanol was added and the mixture was centrifuged at 15,000 rpm for 10 minutes (ethanol rinse), and at this time only the precipitates were kept and the solution was discarded. To the remaining precipitates, reagents were added as shown in Table 16 and the mixture was allowed to stand at room temperature for 15 minutes, centrifuged at 15,000 rpm for 10 minutes, and the solution was discarded while the precipitates were kept. To the remaining precipitates, 500 µl of 70% ethanol was added, the mixture was centrifuged at 15,000 rpm for 10 minutes and the solution was discarded. Finally the precipitates were air dried and dissolved in 22 µl of distilled water.

### (iv) cRNA synthesis: in vitro transcription (second round)

cRNA was synthesized by the similar method used in (ii) from the 22 µl cDNA solution prepared in (iii). However, at the last step, RNA was dissolved in 10 µl of distilled water (RNA content was 5 µg-10 µg).

### (v) Reverse transcription reaction (1^{st} strand cDNA synthesis)

One µl of 0.5 µg/µl random hexamer (1 µg) was added to 10 µl of cRNA solution prepared in (iv), and the mixture was incubated at 65°C for 10 minutes. Then, after rapidly cooling the mixture by placing on ice for 2 minutes or longer, reagents were added as shown in Table 12 and the mixture was incubated at 37°C for 2 minutes so that efficient reverse transcription reaction can be carried out. Then, 1 µl of Superscript II RT was added and the mixture was incubated at 37°C for 1 hour. Subsequently, RNA was removed by adding 1 µl of RNase H. The mixture was incubated at 37°C for 20 minutes, then at 95°C for 2 minutes to separate RNA and DNA and thereafter rapidly cooled by placing on ice for 2 minutes or longer. About 20 µl of the reaction solution described above was mixed with 20 µl of purified water, and 1 µl of the mixture was used as a template for PCR. The condition for PCR and electrophoresis of the products were similar to Example 3 (2) and (3).

### (3) Experimental results

All the stool samples from 7 healthy subjects gave negative results for the five genes, while the stool samples from 25 colon cancer patients were positive as,a whole in about 50% (12/25) for at least one gene. For the cases in which actin mRNA was detected, indicating there were many cells, at least one gene was positive in about 80% (7/9) (Fig. 2). That is, the positive predictive value of this method is 100%, and is far superior to that of the occult blood test for stool (about 0.1%).

### Example 7

### High sensitivity chip analysis using cells isolated from stool of colon cancer patient

Expression analyses by DNA microarray were carried out for cDNA solution samples synthesized in Example 6 after multiplex PCR amplification by incorporated label. Similar to Example 6, 7 healthy subjects and 25 colon cancer patients, total 32 cases were analyzed.

In this Example, the five genes selected in Example 6 were targeted for detection, and multiplex PCR was carried out by adding all 5 kinds of primers to one PCR tube. Similar to Example 4, Cy3-dUTP was added as a substrate to label PCR products. The composition of PCR reaction mixture is shown in Table 20.

**[Table 20]**

| Reaction mixture composition | |
|---|---|
| Component | Composition |
| Invitrogen, AccuPrimer Taq | 1.0 µl |
| 10× AccuPrime PCR Buffer | 2.5 µl |
| Template DNA (cDNA solution) | 1.0 µl |
| Forward Primer (F) | 6.25 pmol/each |
| Reverse Primer (R) | 6.25 pmol/each |
| Cy3 dUTP (1.0 mM, Amersham Biosciences) | 1.0 µl (40 µM) |
| Distilled water | Optional |
| Total | 25 µl |

PCR amplification was carried out for prepared reaction mixtures using a commercially available thermal cycler according to the temperature cycle protocol of Table 4, described in Example 2. However, the cycle number was 35, and the reaction mixture was stored at 4°C after the reaction.

After the reaction, the reaction product was purified using a purification column (QIAGEN, QIAquick PCR Purification Kit) and then made into labeled sample.

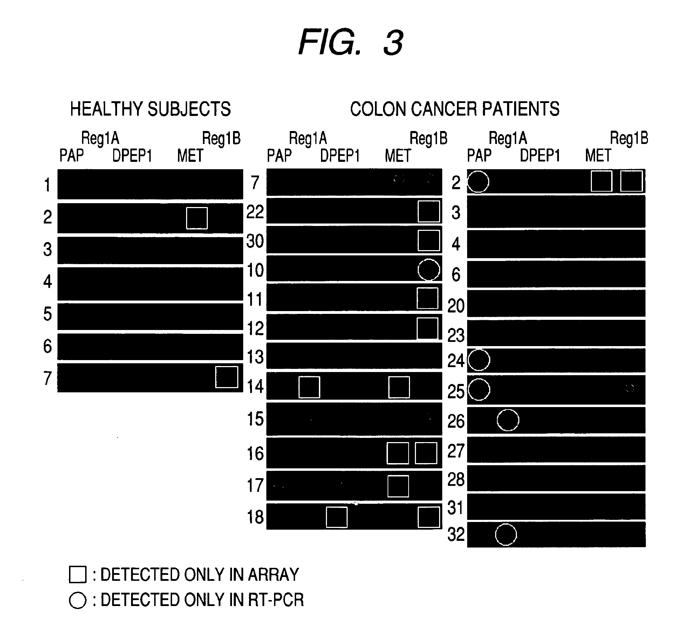

Hybridization on a microarray was carried out according to the method shown in Example 4 using a DNA microarray prepared in Example 4 I and these 32 kinds of labeled samples. The hybridization images obtained as a result are shown in Fig. 3 and fluoroluminescence values to each probe are also shown in Table 21.

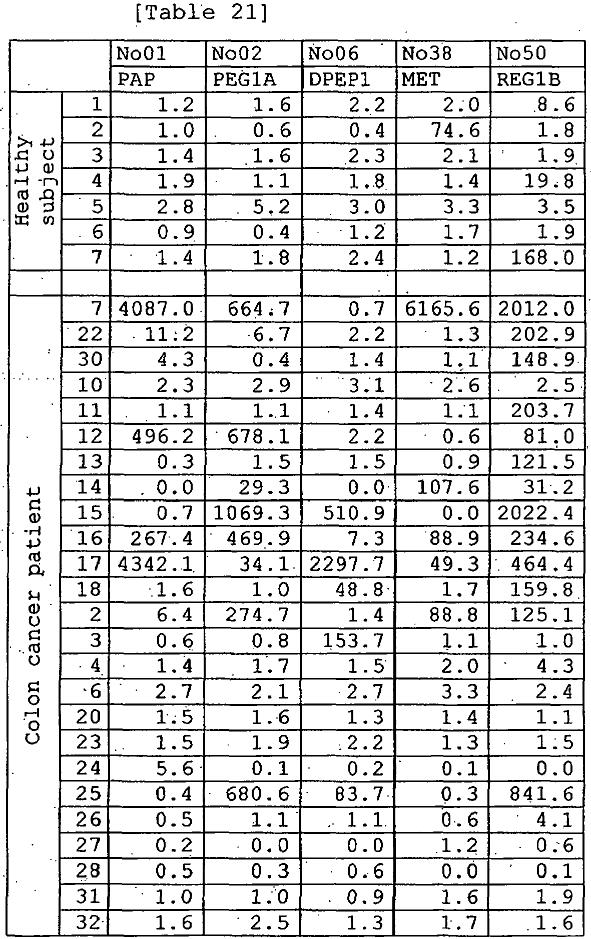

### (3) Experimental results

Table 22 summarizes the presence/absence of the expression of the 5 genes in each case. In fluoroluminescence values in Table 21, a value of 25 or above was defined as positive. If one gene or more among the 5 genes was positive, the judgment was "o". The presence/absence of the expression of β-actin was based on the result of PCR in Example 6. Also, cytodiagnosis in the far right column of the table was the results of the cytodiagnosis for cells recovered from stool samples.

The results for the 5 genes indicated that one gene among the 5 genes was positive in 2 stool samples among 7 stool samples from healthy subjects (false positive 2/7). On the other hand, in 25 stool samples from colon cancer patients, the positivity rate was 56% (14/25) as a whole. For the cases in which actin mRNA was detected, indicating there were many cells, at least one gene was positive in about 90% (8/9). In the cytodiagnosis 6/25 were positive, and in comparison with this result, the result of the expression analysis using the gene set of the present invention would be significant data. Also the correct rate in the positive cases is high at about 90% (14/16), and is superior to that of the occult blood test for stool.

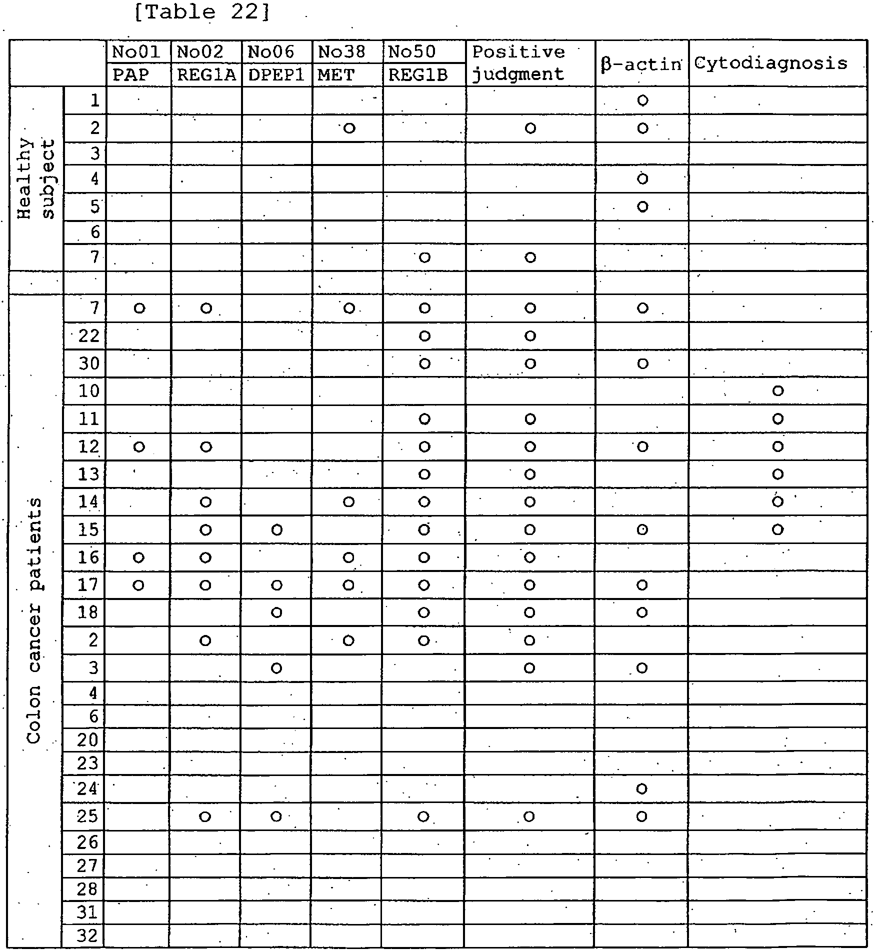

Comparison of the results of the cytodiagnosis with the expression analysis by microarray is shown in Table 23. Among the 6 cases of cytodiagnosis positive, 5 cases are also detected by microarray. Further, among the 19 cases of cytodiagnosis negative, 9 cases, about 50%, can be diagnosed to be positive.

The results clearly demonstrated that colon cancer diagnosis can be possible using small amount of cells recovered from stool by preparing the sample by multiplex PCR and analyzing by microarray.

**[Table 23]**

| Comparison with cytodiagnosis results | | | |
|---|---|---|---|
| Cancer patient | - (negative) | 9/19 (47%) | 14/25 (56%) |
| | + (positive) | 5/6 (83%) | |
| Healthy subject | | | 2/7 (28%) |

Further, the results of the analysis by the microarray of the present Example are superior in sensitivity compared to the results of RT-PCR shown in Example 6, and total 15 spots among the 32 samples were rescued. On the other hand, 6 spots could be detected by RT-PCR but not by microarray due to poor PCR amplification because it was multiplex PCR (Fig. 3). Combining the merits of the both methods, the presence/absence of the expression of the 5 genes in each case is summarized in Table 24. When the result of either RT-PCR or microarray was positive, the positive judgment "o" was given. Also, the positivity rate was calculated from this result and compared with the cytodiagnosis results (Table 25). By combining the detection results of RT-PCR and microarray as shown in Tables 24 and 25, the positivity rate of colon cancer patients was 72% (18/25) confirming that the gene set of the present invention is efficacious for the diagnosis of colon cancer.

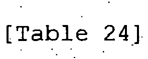

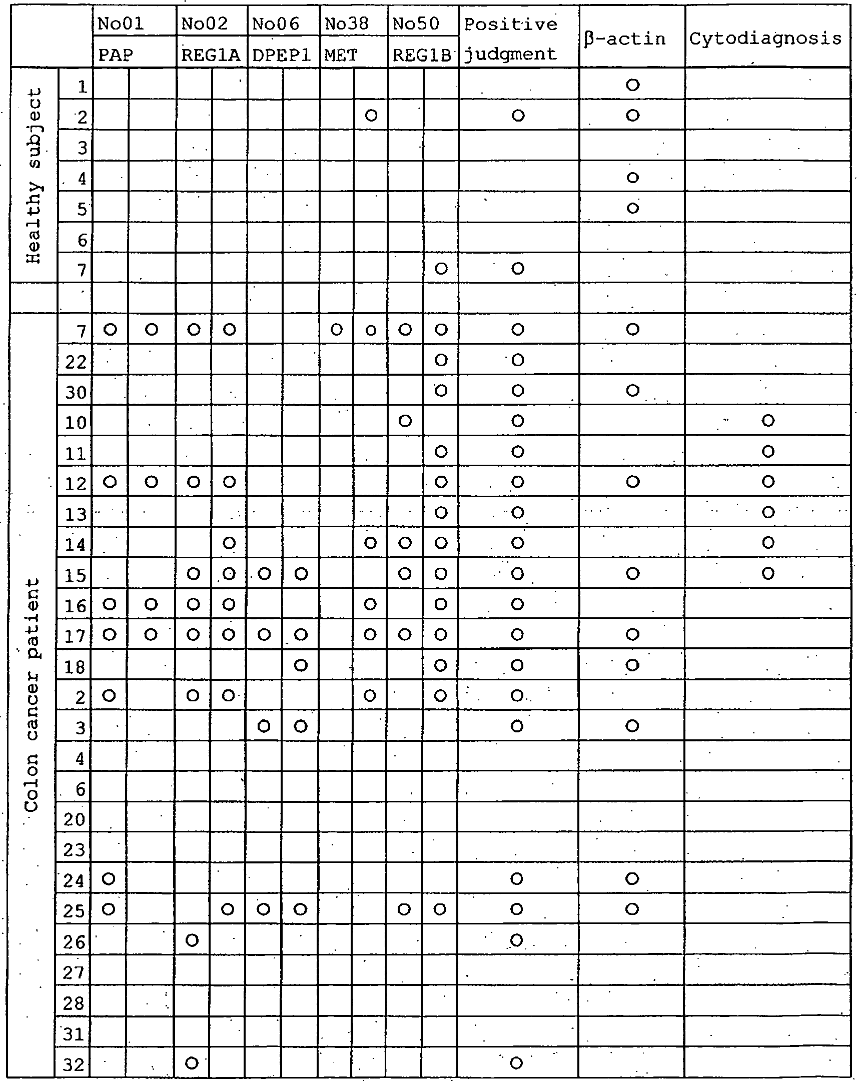

**[Table 25]**

| Comparison with cytodiagnosis results | | | |
|---|---|---|---|
| Cancer patient | - (negative) | 12/19 (63%) | 18/25 (72%) |
| | + (positive) | 6/6 (100%) | |
| Healthy subject | | | 2/7 (28%) |

**[Table 26]**

| No. | Gene name | probe (5'→3') | Sequence ID No. |
|---|---|---|---|
| 1 | PAP | TTCCCCCAACCTGACCACCTCATTCTTATCTTTCTTCTGTTTCTTCCTCCCCGCTGTCAT | SEQ ID NO: 1 |
| 2 | REG1A | GACCATCTCTCCAACTCAACTCAACCTGGACACTCTCTTCTCTGCTGAGTTTGCCTTGTT | SEQ ID NO: 2 |
| 6 | DPEP1 | CAGATGCCAGGAGCCCTGCTGCCCACATGCAAGGACCAGCATCTCCTGAGAG | SEQ ID NO: 6 |
| 8 | TACSTD2 | ATCTGTATGACAACCCGGGATCGTTTGCAAGTAACTGAATCCATTGCGACATTGTGAAGG | SEQ ID NO: 8 |
| 10 | SLCO4A1 | CAGCATTCCTGCACTAACGGCAACTCTACGATGTGTCCGTGACCCTCAGAGATC | SEQ ID NO: 10 |
| 11 | SFRP4 | ACAAACCCGAAAAGAGTGTGAGCTAACTAGTTTCCAAAGCGGAGACTTCCGACTTCCTTA | SEQ ID NO: 11 |
| 25 | NQO1 | GTCTTAGAACCTCAACTGACATATAGCATTGGGCACACTCCAGCAGACGCCCGAATTCAA | SEQ ID NO: 25 |
| 30 | AURKB | TATGTCTGTGTATGTATAGGGGAAAGAAGGGATCCCTAACTGTTCCCTTATCTGTTTTCT | SEQ ID NO: 30 |
| 37 | ITGB4 | GGAGGACTACGACAGCTTCCTTATGTACAGCGATGACGTTCTACGCTCTCCATCGG | SEQ ID NO: 37 |
| 38 | MET | CTGCCTGACCTTTAAAAGGCCATCGATATTCTTTGCTCCTTGCCATAGGACTTGTATTGT | SEQ ID NO: 38 |
| 40 | PROX1 | CAGCACCGCCGAAGGGCTCTCCTTGTCGCTCATAAAGTCCGAGTGCGGCGATCTTCAAGA | SEQ ID NO: 40 |
| 41 | WTAP | GGAAGTTTACGCCTGATAGCCAAACAGGGAAAAAGTTAATGGCGAAGTGTCGAATGCTTA | SEQ ID NO: 41 |
| 42 | FLJ10858 | AAAGGCCGGATGCTAGGTGATGTGCTAATGGATCAGAACGTATTGCCTGGAGTAGGGAAC | SEQ ID NO: 42 |
| 46 | TRIM31 | CTCAGGATACGAAGACATTTGACGTTGCGCTGTCCGAGGAGCTCCATGCGGCAC | SEQ ID NO: 46 |
| 50 | REG1B | AACTGGTCCTGCAATTACTATGAAGTCAAAAATTAAACTAGACTATGTCTCCAACTCAGT | SEQ ID NO: 50 |

**[Table 27]**

| No. | Gene name | Forward-Primer(5'→3') | Sequence ID No. | Reverse-Primer(5'-3') | Sequence ID No. |
|---|---|---|---|---|---|
| 1 | PAP | GAGAAGCACAGCATTTCTGAG | SEQ ID NO: 51 | TGCTCTTTAAAGCCTTAGGCC | SEQ ID NO: 52 |
| 2 | REG1A | AATCCTGGCTACTGTGTGAG | SEQ ID NO: 53 | TCCAAAGACTGGGGTAGGT | SEQ ID NO: 54 |
| 6 | DPEP1 | ACCCATTACGGCTACTCCTC | SEQ ID NO: 61 | AAGGGGTGTTGCTTTTATTGC | SEQ ID NO: 62 |
| 8 | TACSTD2 | GAGAAAGGAACCGAGCTTGT | SEQ ID NO: 65 | TGGTAGTAAGGGCAAGCTGA | SEQ ID NO: 66 |
| 10 | SLCO4A1 | GAAGGCCACCTGAACCTAAC | SEQ ID NO: 69 | CCATCTGAAGACTCCGACAG | SEQ ID NO: 70 |
| 11 | SFRP4 | GATCTTCAAGTCCTCATCACC | SEQ ID NO: 71 | ACCAGCTTTAACTCACCTTC | SEQ ID NO: 72 |
| 25 | NQO1 | CGCAGACCTTGTGATATTCC | SEQ ID NO: 99 | CGATTCCCTCTCATTTATTCCTT | SEQ ID NO: 100 |
| 30 | AURKB | ACCTCATCTCCAAACTGCTCA | SEQ ID NO: 109 | AAAAAGCTTCAGCCTTTATTAAACA | SEQ ID NO: 110 |
| 37 | ITGB4 | TCTGGCCTTCAATGTCGTCT | SEQ ID NO: 123 | TGTGGTCGAGTGTGAGTGTT | SEQ ID NO: 124 |
| 38 | MET | ATGTCCATGTGAACGCTACT | SEQ ID NO: 125 | CCAAGCCTCTGGTTCTGATG | SEQ ID NO: 126 |
| 40 | PROX1 | TGATGGCCTATCCATTTCAG | SEQ ID NO: 129 | AACATCTTTGCCTGCGATAA | SEQ ID NO: 130 |
| 41 | WTAP | AAGCAACAACAGCAGGAGTC | SEQ ID NO: 131 | TGTGAAATCCAGACCCAGAC | SEQ ID NO: 132 |
| 42 | FLJ10858 | ATTTCGGAATGAAAGGCTTC | SEQ ID NO: 133 | CCCTGCTAGATGTCCAACTG | SEQ ID NO: 134 |
| 46 | TRIM31 | TGTTCCTCTGGAACTGGAGA | SEQ ID NO: 141 | CCCTCCTTTTGCTCAAGAAT | SEQ ID NO: 142 |
| 50 | REG1B | CTCAGGATTCAAGAAATGGAAGG | SEQ ID NO: 149 | GTGAAGGTACTGAAGATCAGCG | SEQ ID NO: 150 |

**[Table 28]**

| No. | Gene name | probe (5'→3') | Sequence ID No. |
|---|---|---|---|
| 1 | PAP | TTCCCCCAACCTGACCACCTCATTCTTATCTTTCTTCTGTTTCTTCCTCCCCGCTGTCAT | SEQ ID NO: 1 |
| 2 | REG1A | GACCATCTCTCCAACTCAACTCAACCTGGACACTCTCTTCTCTGCTGAGTTTGCCTTGTT | SEQ ID NO: 2 |
| 6 | DPEP1 | CAGATGCCAGGAGCCCTGCTGCCCACATGCAAGGACCAGCATCTCCTGAGAG | SEQ ID NO: 6 |
| 38 | MET | CTGCCTGACCTTTAAAAGGCCATCGATATTCTTTGCTCCTTGCCATAGGACTTGTATTGT | SEQ ID NO: 38 |
| 50 | REG1B | AACTGGTCCTGCAATTACTATGAAGTCAAAAATTAAACTAGACTATGTCTCCAACTCAGT | SEQ ID NO: 50 |

**[Table 29]**

| No. | Gene name | Forward-Primer(5'→3') | Sequence ID No. | Reverse-Primer (5'→3') | Sequence ID No. |
|---|---|---|---|---|---|
| 1 | PAP | GAGAAGCACAGCATTTCTGAG | SEQ ID NO: 51 | TGCTCTTTAAAGCCTTAGGCC | SEQ ID NO: 52 |
| 2 | REG1A | AATCCTGGCTACTGTGTGAG | SEQ ID NO: 53 | TCCAAAGACTGGGGTAGGT | SEQ ID NO: 54 |
| 6 | DPEP1 | ACCCATTACGGCTACTCCTC | SEQ ID NO: 61 | AAGGGGTGTTGCTTTTATTGC | SEQ ID NO: 62 |
| 38 | MET | ATGTCCATGTGAACGCTACT | SEQ ID NO: 125 | CCAAGCCTCTGGTTCTGATG | SEQ ID NO: 126 |
| 50 | REG1B | CTCAGGATTCAAGAAATGGAAGG | SEQ ID NO: 149 | GTGAAGGTACTGAAGATCAGCG | SEQ ID NO: 150 |

### Example 8

### Selection of the marker genes for colon cancer screening using stool (obtaining the expression profiles of about 39000 genes by microarray, and selection of the marker genes)

Genome-wide gene expression analyses were carried out targeting the total RNA extracted in Example 6 (1) using a microarray (human U133 oligonucleotide probe arrays (Affymetric, USA)). Experiments were carried out according to the method recommended by the manufacturer (see Example 1 (2)). The targets used were 4 kinds of cell RNA isolated from stool samples of colon cancer patients and a mixture of 7 kinds of cell RNA isolated from stool samples of healthy subjects, total 5 kinds of RNA.

Signals obtained by the hybridization between the microarray and the targets were read using a GeneArray scanner (Affymetrix), and the intensity was analyzed using a computer software, Microarray Suite 5.0 (Affymetrix). Amount of the gene expression was analyzed using Microsoft Excel, and the genes were chosen which were expressed at high level in all the cases of colon cancer described above but not detected in healthy subjects and in peripheral blood (result of Example 1). As the result, 7 kinds of genes were chosen (Table 30). All of the 7 genes fulfilled the conditions for the screening markers for colon cancer using stool as samples , which were proposed by the present inventors. Specific probes and primers were designed as shown in Table 30 and Table 31 for these 7 genes.

**[Table 30]**

| No. | Gene name | GenBank ID | probe (5'→3') | Sequence ID No. |
|---|---|---|---|---|
| 51 | SEPP1 | NM_005410 | CCATAGTCAATGATGGTTTAATAGGTAAACCAAACCCTATAAACCTGACCTCCTTTATGG | SEQ ID NO: 151 |
| 52 | RPL27A | NM_000990 | CCAACTGTCAACCTTGACAAATTGTGGACTTTGGTCAGTGAACAGACACGGGTGAATGCT | SEQ ID NO: 152 |
| 53 | ATP1B1 | NM_001677 | GAGTGTAAGGCGTACGGTGAGAACATTGGGTACAGTGAGAAAGACCGTTTTCAGGGAGCT | SEQ ID NO: 153 |
| 54 | EEF1A1 | NM_001402 | CCACCCCACTCTTAATCAGTGGTGGAAGAACGGTTTCAGAACTGTTTGTTTCAATTGGCC | SEQ ID NO: 154 |
| 55 | SFN | NM_006142 | CTCTGATCGTAGGAATTGAGGAGTGTCCCGCCTTGTGGCTGAGAACTGGACAGTGG | SEQ ID NO: 155 |
| 56 | RPS11 | NM_001015 | TCATCCGCCGAGACTATCTGCACTACATCCGCAAGTACAACCGCTTCGAGAAGCG | SEQ ID NO: 156 |
| 57 | RPL23 | NM_000978 | ACATCCAGCAGTGGTCATTCGACAACGAAAGTCATACCGTAGAAAAGATGGCGTGTTTCT | SEQ ID NO: 157 |

**[Table 31]**

| No. | Gene name | Forward-Primer (5'→3') | Sequence ID No. | Reverse-Primer (5'→3') | Sequence ID No. |
|---|---|---|---|---|---|
| 51 | SEPP1 | AATTAGCAGTTTAGAATGGAGG | SEQ ID NO: 158 | CTGTATCCAATTCTGTACTGC | SEQ ID NO: 165 |
| 52 | RPL27A | TGGGCTGCCAACATGCCATC | SEQ ID NO: 159 | TGTAGTAGCCCGATCGCACC | SEQ ID NO: 166 |
| 53 | ATPIB1 | GGCAAGCGAGATGAAGATAAGG | SEQ ID NO: 160 | AGGTCCCATA CGTATGACAG | SEQ ID NO: 167 |
| 54 | EEF1A1 | AGACTATCCACCTTTGGGTCG | SEQ ID NO: 161 | GATGCATTGTTATCATTAACCAGTC | SEQ ID NO: 168 |
| 55 | SFN | TTGAGCGCACCTAACCACTGGT | SEQ ID NO: 162 | GAGAGGAAACATGGTCACACCCA | SEQ ID NO: 169 |
| 56 | RPS11 | ACATTCAGACTGAGCGTGCCTA | SEQ ID NO: 163 | GATCTGGACGTCCCTGAAGCA | SEQ ID NO: 170 |
| 57 | RPL23 | TTCAAGATGTCGAAGCGAGGAC | SEQ ID NO: 164 | TGTAATGGCAGAACCTTTCATCTCG | SEQ ID NO: 171 |

### Example 9

### RT-PCR and high sensitivity chip analyses using cells isolated from stool samples of colon cancer patients

### RT-PCR analyses

RT-PCR analyses were carried out for the 7 genes chosen in Example 8 using cell RNA isolated from 7 healthy subjects and 25 colon cancer patients. The 7 genes described above, their probes and primers are summarized in Table 30 and 31. The experimental procedures are the same as in Example 6 (i)-(v).

The results of the analysis for the 7 genes indicated that in the stool samples from 7 healthy subjects' only 1 case was positive for 1 gene (No. 102). On the other hand, in the stool samples from 25 colon cancer patients, as a whole 64% (16/25) was positive for at least 1 gene. For the 9 cases in which β-actin mRNA was detected, indicating there were many cells, at least one gene was positive in about 90% (8/9) (Table 32).

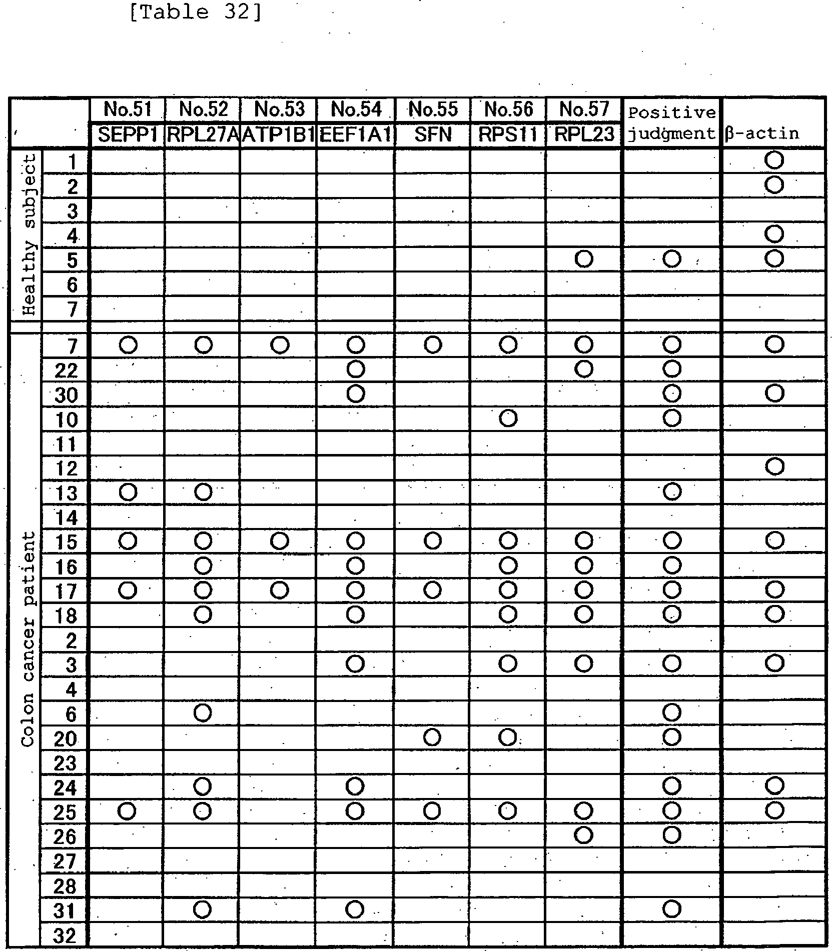

### (2) Chip analysis

Probe DNAs (SEQ ID NO: 151-157) for detecting the 7 genes chosen in Example 8 were synthesized and DNA microarrays were prepared as described in Example 4 I. Using this DNA microarray, expression analysis was carried out in a similar manner to Example 7 after performing multiplex PCR amplification by incorporated label, the targets of which are the 7 genes, using primers shown in Table 31. The multiplex PCR was performed by adding all the 7 kinds of primers to a PCR tube. The substrates were mixed with Cy3-dUTP to standardize PCR products. The PCR reaction mixture composition, temperature cycle protocol and method for hybridizing to the DNA microarray are similar to those in Example 7. Fluorescent luminescence values of each probe to the 32 labeled samples are shown in Table 33.

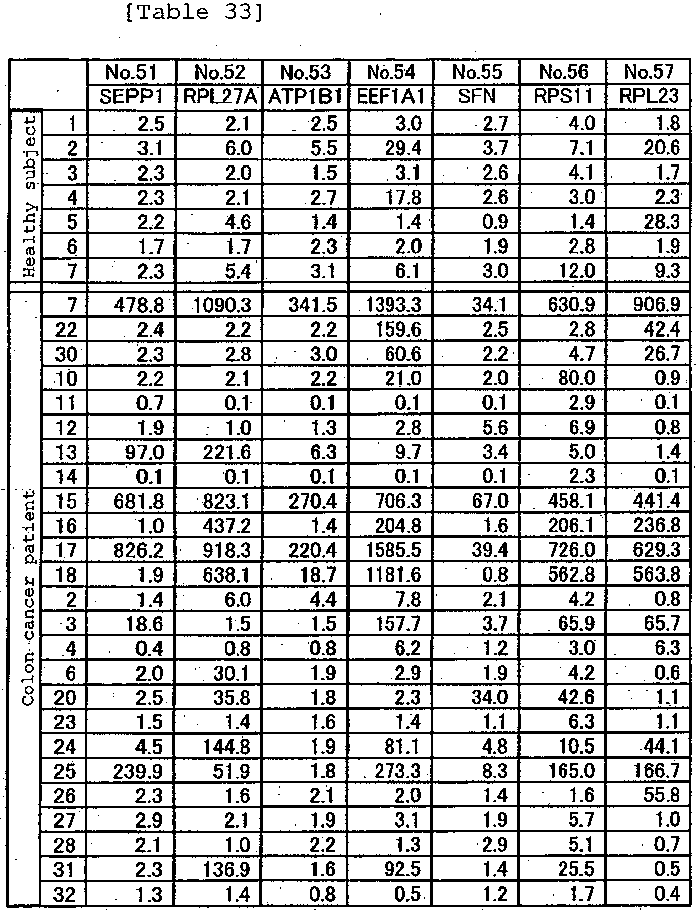

### (3) Experimental results

The presence/absence of the expression of the 7 genes in each case is summarized in Table 34. In fluoroluminance values in Table 33, a value of 30 or above was defined as positive. If one gene or more among the 7 genes was positive, the judgment was "○". The presence/absence of the expression of β-actin and cytodiagnosis are the same as Table 22 in Example 7.

The results for the 7 genes indicated that all of the 7 stool samples from healthy subjects were negative. On the other hand, in 25 stool samples from colon cancer patients, the positivity rate was 64% (16/25) as a whole. For the cases in which actin mRNA was detected, indicating there were many cells, at least one gene was positive in about 90% (8/9). In the cytodiagnosis 6/25 were positive, and in comparison with this result, the result of the expression analysis using the 7 genes set of the present invention would be significant data. Also the positive predictive rate is 100% and is superior to that of the occult blood test for stool.

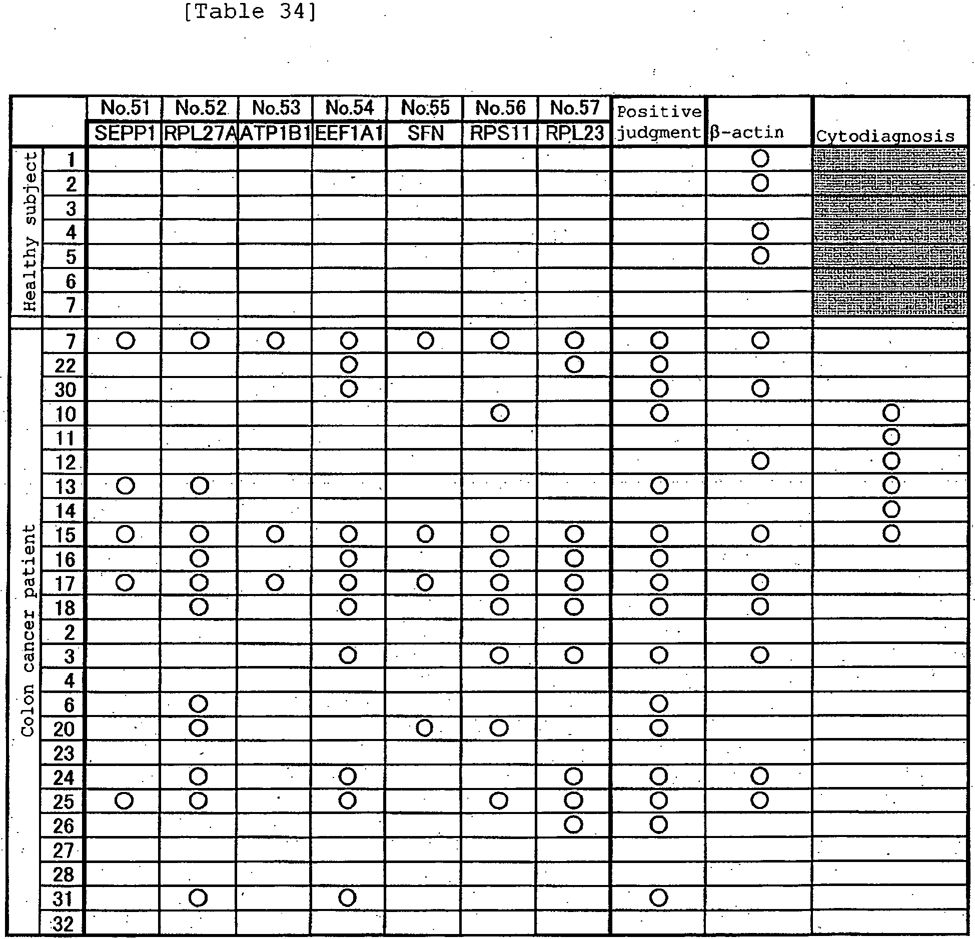

The results of Table 32 and Table 34 indicated that the results of RT-PCR of (1) and DNA microarray analysis of (2) were almost the same. Thus, it has been shown that diagnosis of colon cancer is possible by preparing samples by multiplex PCR and analyzing by microarray using small number of cells recovered from stool.

Further, the results combined with the results shown in Example 7 are shown in Table 35. Here, the targeting genes were 11 genes because the No. 38 gene with relatively low detection rate was eliminated. The results of the analysis for the 11 genes indicated that in the stool samples from 7 healthy subjects only 1 case was positive for 1 gene. On the other hand, in the stool samples from 25 colon cancer patients, 20 cases were positive for at least 1 gene, and the positivity rate was 80% (20/25). For the cases in which β-actin mRNA was detected; indicating there were many cells, were positive in 100% (9/9).

Table 36 shows comparison of the results of cytodiagnosis with that of the expression analysis by microarray. The 6 cytodiagnosis positive cases were all detected by microarray, too. Further, for the 19 cytodiagnosis negative cases, 14 cases, which was about 70%, could be diagnosed as positive, indicating that the results by microarray was far superior to that by cytodiagnosis. As described above, it has been confirmed that the gene set of the present invention is effective for diagnosis of colon cancer.

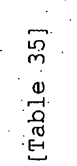

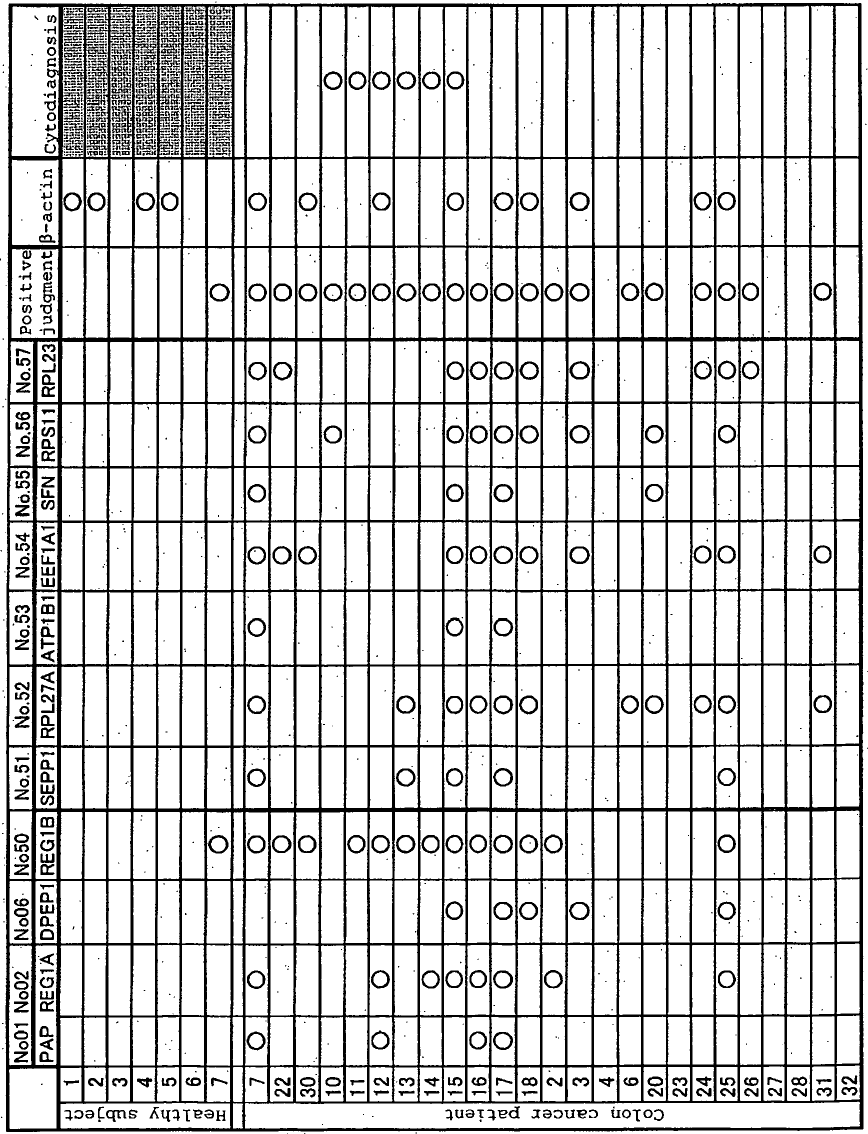

**[Table 36]**

| Comparison with cytodiagnosis results | | | |
|---|---|---|---|
| Cancer patient | - (negative) | 14/19(74%) | 20/25(80%) |
| | + (positive) | 6/6(100%) | |
| Healthy subject | | | 1/7(14%) |

The 57 genes of the present invention are useful as diagnostic markers for colon cancer. Thus, the probes, primers and samples fixed to solid phase of the present invention can be utilized for early diagnosis of colon cancer.

The present invention is not limited to the above embodiments and various changes and modifications can be made within the spirit and scope of the present invention. Therefore to apprise the public of the scope of the present invention, the following claims are made.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A method for screening colon cancer cells in a sample derived from a subject to be examined, comprising measuring an amount of a mRNA of two or more genes selected from the group of genes Nos. 1 to 50 in Table 1,
wherein the mRNA is hybridizable specifically with two or more probes comprising an oligonucleotide having a base sequence selected from the sequences of SEQ ID NOs: 1, 2, 6, 8, 10, 11, 25, 30, 37, 38, 40, 41, 42, 46 and 50; and
wherein the sample is a smear of stool.

2. The method according to claim 1, wherein the two or more probes each comprise an oligonucleotide having a base sequence selected from the sequences of SEQ ID NOs: 1, 2, 6, 38 and 50.

3. The method according to claim 1 or 2, wherein the smear of stool is naturally excreted.

4. The method according to claim 1, wherein the method further comprises concluding that the sample derived from the subject to be examined contains colon cancer cells when the measured amount of the mRNA in the sample is higher than the measured amount of the same mRNA in a sample derived from a healthy subject.

5. A method for exhibiting the presence of colon cancer cells in a sample by using the method according to any of claims 1 to 4.

6. The method according to claim 5, wherein the colon cancer is early colon cancer.

7. Use of a primer in the method according to any of claims 1 to 6 for amplifying specifically a target gene listed in Table 1, said primer comprising an oligonucleotide having a base sequence represented by any one of SEQ ID NOs: 51 to 150.

8. Use of a probe in the method according to any of claims 1 to 6 for detecting a target gene listed in Table 1, said probe being hybridizable specifically with the target gene and comprising an oligonucleotide having any one of the base sequences of SEQ ID NOs: 1, 2, 6, 8, 10, 11, 25, 30, 37, 38, 40, 41, 42, 46 and 50.

9. Use of a solid-phase test material, wherein a probe as defined in claim 8 is immobilized on a solid-phase carrier, for detecting colon cancer cells based on a smear of stool as sample.

10. Use of a kit for detecting colon cancer cells based on a smear of stool as sample, said kit comprising:
a primer as defined in claim 7 for amplifying specifically a target gene listed in Table 1; and
a probe as defined in claim 8 or a solid-phase test material as defined in claim 9.

## Patentansprüche

1. Verfahren zum Prüfen auf Kolonkrebszellen in einer von einem zu untersuchenden Subjekt stammenden Probe,
welches Messen einer Menge an einer mRNA von zwei oder mehr Genen, ausgewählt aus der Gruppe von Genen mit Nrn. 1 bis 50 in Tabelle 1, umfasst,
wobei die mRNA spezifisch mit zwei oder mehr Sonden hybridisierbar ist, die ein Oligonukleotid umfassen mit einer Basensequenz, ausgewählt aus den Sequenzen mit Seq.-Id.-Nrn. 1, 2, 6, 8, 10, 11, 25, 30, 37, 38, 40, 41, 42, 46 und 50; und
wobei die Probe ein Stuhlabstrich ist.

2. Verfahren nach Anspruch 1, bei dem die zwei oder mehr Sonden jeweils ein Oligonukleotid mit einer aus den Sequenzen mit Seq.-Id.-Nrn. 1, 2, 6, 38 und 50 ausgewählten Basensequenz umfassen.

3. Verfahren nach Anspruch 1 oder 2, bei dem der Stuhlabstrich natürlich ausgeschieden ist.

4. Verfahren nach Anspruch 1, bei dem das Verfahren weiterhin ein Konkludieren umfasst, dass die von dem zu untersuchenden Subjekt stammende Probe Kolonkrebszellen enthält, wenn die gemessene Menge an der mRNA in der Probe höher ist als die gemessene Menge an derselben mRNA in einer von einem gesunden Subjekt stammenden Probe.

5. Verfahren zum Aufzeigen des Vorhandenseins von Kolonkrebszellen in einer Probe durch Benutzen des Verfahrens nach einem der Ansprüche 1 bis 4.

6. Verfahren nach Anspruch 5, bei dem der Kolonkrebs Kolonfrühkrebs ist.

7. Benutzen eines Primers im Verfahren nach einem der Ansprüche 1 bis 6 zum Amplifizieren spezifisch eines in Tabelle 1 aufgelisteten Zielgens, wobei besagter Primer ein Oligonukleotid mit einer durch eine von Seq.-Id.-Nrn. 51 bis 150 dargestellten Basensequenz umfasst.

8. Benutzen einer Sonde im Verfahren nach einem der Ansprüche 1 bis 6 zum Nachweisen eines in Tabelle 1 aufgelisteten Zielgens, wobei besagte Sonde spezifisch mit dem Zielgen hybridisierbar ist und ein Oligonukleotid mit einer der Basensequenzen aus Seq.-Id.-Nrn. 1, 2, 6, 8, 10, 11, 25, 30, 37, 38, 40, 41, 42, 46 und 50 umfasst.

9. Benutzen eines Festphasentestmaterials, bei dem eine wie in Anspruch 8 definierte Sonde auf einem Festphasenträger immobilisiert ist, zum Nachweisen von Kolonkrebszellen basierend auf einem Stuhlabstrich als Probe.

10. Benutzen eines Kits zum Nachweisen von Kolonkrebszellen basierend auf einem Stuhlabstrich als Probe, wobei besagtes Kit umfasst:
einen wie in Anspruch 7 definierten Primer zum Amplifizieren spezifisch eines in Tabelle 1 aufgelisteten Zielgens; sowie
eine wie in Anspruch 8 definierte Sonde oder ein wie in Anspruch 9 definiertes Festphasentestmaterial.

## Revendications

1. Procédé pour dépister des cellules de cancer du côlon dans un échantillon provenant d'un sujet à examiner, comprenant la mesure d'une quantité d'un ARNm de deux ou plus de deux gènes choisis dans le groupe de gènes N°^{s} 1 à 50 sur le Tableau 1,
dans lequel l'ARNm est hybridable spécifiquement avec deux ou plus de deux sondes comprenant un oligonucléotide ayant une séquence de bases choisie parmi les séquences des SEQ ID Nos : 1, 2, 6, 8, 10, 11, 25, 30, 37, 38, 40, 41, 42, 46 et 50 ; et
dans lequel l'échantillon est un frottis de selles.

2. Procédé suivant la revendication 1, dans lequel les deux ou plus de deux sondes comprennent chacune un oligonucléotide ayant une séquence de bases choisie parmi les séquences des SEQ ID Nos : 1, 2, 6, 38 et 50.

3. Procédé suivant la revendication 1 ou 2, dans lequel le frottis de selles est excrété naturellement.

4. Procédé suivant la revendication 1, ledit procédé comprenant en outre la conclusion que l'échantillon provenant du sujet à examiner contient des cellules de cancer du côlon lorsque la quantité mesurée de l'ARNm dans l'échantillon est supérieure à la quantité mesurée du même ARNm dans un échantillon provenant d'un sujet sain.

5. Procédé pour mettre en évidence la présence de cellules de cancer du côlon dans un échantillon en utilisant le procédé suivant l'une quelconque des revendications 1 à 4.

6. Procédé suivant la revendication 5, dans lequel le cancer du côlon est un cancer précoce du côlon.

7. Utilisation d'une amorce dans le procédé suivant l'une quelconque des revendications 1 à 6 pour amplifier spécifiquement un gène cible indiqué sur le Tableau 1, ladite amorce comprenant un oligonucléotide et une séquence de bases représentée par l'une quelconque des SEQ ID Nos : 51 à 150.

8. Utilisation d'une sonde dans le procédé suivant l'une quelconque des revendications 1 à 6 pour la détection d'un gène cible indiqué sur le Tableau 1, ladite sonde étant hybridable spécifiquement avec le gène cible et comprenant un oligonucléotide ayant l'une quelconque des séquences de bases des SEQ ID Nos : 1, 2, 6, 8, 10, 11, 25, 30, 37, 38, 40, 41, 42, 46 et 50.

9. Utilisation d'une substance d'essai en phase solide, dans laquelle une sonde telle que définie dans la revendication 8 a été immobilisée sur un support en phase solide, pour la détection de cellules de cancer du côlon d'après un frottis de selles comme échantillon.

10. Utilisation d'un kit pour la détection de cellules de cancer du côlon d'après un frottis de selles comme échantillon, ledit kit comprenant :
une amorce telle que définie dans la revendication 7 pour amplifier spécifiquement un gène cible indiqué sur le Tableau 1 ; et
une sonde telle que définie dans la revendication 8 ou une substance d'essai en phase solide telle que définie dans la revendication 9.
